# EUROPEAN PATENT SPECIFICATION

(11) **EP 2 768 507 B1**
(45) Date of publication and mention of the grant of the patent: **11.12.2019**
(21) Application number: 12797979.7
(22) Date of filing: 18.10.2012
(51) Int. Cl.: A61K 31/439, A61P 25/28, C12Q 1/6883

(54) **BIOMARKERS PREDICTIVE OF RESPONSIVENESS TO ALPHA 7 NICOTINIC ACETYLCHOLINE RECEPTOR ACTIVATOR TREATMENT**
PRÄDIKTIVE BIOMARKER DES ANSPRECHENS AUF EINE BEHANDLUNG MIT DEM NIKOTINISCHEN ACETYLCHOLINREZEPTOR ALPHA-7
BIOMARQUEURS PRÉDICTIFS DE LA SENSIBILITÉ AU TRAITEMENT PAR L'ACTIVATEUR DU RÉCEPTEUR DE L'ACÉTYLCHOLINE NICOTINIQUE ALPHA 7

(30) Priority: 20.10.2011 US 201161549319 P
(43) Date of publication of application: 27.08.2014
(73) Proprietor: Novartis AG, 4056 Basel (CH)
(72) Inventor: FEUERBACH, Dominik, 4002 Basel (CH); GOMEZ-MANCILLA, Baltazar, 4002 Basel (CH); HE, Yunsheng, Cambridge, MA 02139 (US); JOHNS, Donald, Cambridge, MA 02139 (US); LOPEZ-LOPEZ, Cristina, 4002 Basel (CH); MCALLISTER, Kevin Hall, 4002 Basel (CH); PEZOUS, Nicole, 4002 Basel (CH); SANDFORD, Lisa, 4002 Basel (CH); WEISS, Markus, 4002 Basel (CH)
(74) Representative: Appleyard Lees IP LLP
(86) International application number: PCT/IB2012/055692
(87) International publication number: WO 2013/057687

(56) References cited:
- ANATOLY A. MAZUROV ET AL: "Discovery and Development of [alpha]7 Nicotinic Acetylcholine Receptor Modulators", JOURNAL OF MEDICINAL CHEMISTRY, vol. 54, no. 23, 15 September 2011 (2011-09-15), pages 7943-7961, XP055060345, ISSN: 0022-2623, DOI: 10.1021/jm2007672
- HORENSTEIN NICOLE A ET AL: "Multiple Pharmacophores for the Selective Activation of Nicotinic alpha 7-Type Acetylcholine Receptors", MOLECULAR PHARMACOLOGY, vol. 74, no. 6, 2 September 2008 (2008-09-02), pages 1496-1511, XP55060348, ISSN: 0026-895X
- L. E. HONG ET AL: "A CHRNA5 allele related to nicotine addiction and schizophrenia", GENES, BRAIN AND BEHAVIOR, vol. 10, no. 5, 6 April 2011 (2011-04-06), pages 530-535, XP055059804, ISSN: 1601-1848, DOI: 10.1111/j.1601-183X.2011.00689.x
- NADINE PETROVSKY ET AL: "Sensorimotor Gating is Associated with CHRNA3 Polymorphisms in Schizophrenia and Healthy Volunteers", NEUROPSYCHOPHARMACOLOGY, vol. 35, no. 7, June 2010 (2010-06), pages 1429-1439, XP055059821, ISSN: 0893-133X, DOI: 10.1038/npp.2010.12
- GEORG WINTERER ET AL: "Risk gene variants for nicotine dependence in the CHRNA5-CHRNA3-CHRNB4 cluster are associated with cognitive performance", AMERICAN JOURNAL OF MEDICAL GENETICS PART B: NEUROPSYCHIATRIC GENETICS, vol. 153B, no. 8, 5 December 2010 (2010-12-05), pages 1448-1458, XP055059810, ISSN: 1552-4841, DOI: 10.1002/ajmg.b.31126
- JOHANSSON I ET AL: "CNVs of human genes and their implication in pharmacogenetics", CYTOGENETIC AND GENOME RESEARCH 200903 CH, vol. 123, no. 1-4, March 2009 (2009-03), pages 195-204, XP9168924, ISSN: 1424-8581, DOI: 10.1159/000184709

## Description

### BACKGROUND OF THE INVENTION

Cognitive deficits have been recognized as a clinically significant aspect of schizophrenia and the major factor determining successful functional rehabilitation and social reintegration (Green, 1996; Green, 2007). A cognitive impairment in schizophrenia or in other mental disease is an acquired deficit in one or more of memory function, problem solving, orientation and/or abstraction that impinges on an individual's ability to function independently, particularly pronounced in verbal memory, executive functions, attention and vigilance, verbal fluency and motor speed, but also including other functions. Cognitive impairments are not the result of positive or negative symptoms of the disorder or accounted for by motivational deficits (Harvey et al, 2004). In most cases cognitive impairments do not worsen or improve with illness progression (Harvey et al, 2004; Hoff et al, 1999). There are no accepted treatments for cognitive deficits in schizophrenia. Currently available antipsychotic treatments do not improve cognition beyond practice effects (Goldberg et al, 2007; Keefe et al, 2007).

Several lines of evidence suggest that the alpha 7 nicotinic acetylcholine receptor (α7-nAChR) could be involved in cognitive dysfunctions in schizophrenia. A link between P50 sensory gating deficits displayed by schizophrenics and a defect on chromosome 15q14, the site of the α7-nAChR gene (Chini et al., 1994), was established by Freedman et al., 1997. Polymorphisms in the α7-nAChR promoter region that decreased transcription were more prevalent in schizophrenic patients than in the control subjects (Leonard et al., 2002). Postmortem studies have demonstrated that α7-nAChR levels are decreased in brains of schizophrenia patient's (Freedman et al., 1995). α7-nAChRs are expressed in key brain areas important for learning and memory (hippocampus, prefrontal cortex and amygdala). Activation of the α7-nAChR has been shown to modulate glutamatergic, GABAergic and cholinergic neurotransmitter release and to improve cognition in a variety of different pre-clinical animal models.

Novel α7-nAChR activators have been developed for the treatment of disease conditions associated with defective or malfunctioning nicotinic acetylcholine receptors. Although it has been assumed that α7-nAChR activator treatment might improve cognitive deficits controversial data and variation of individual cognitive responses to α7-nAChR activator treatments have so far hindered the development of α7-nAChR activator based treatments of cognitive impairments, e.g. it is not known why some patients do not respond to these medications. Consequently, there is a need to predict in advance of treatment whether a patient suffering from cognitive impairments or dysfunctions is likely to be responsive to treatment with an α7-nAChR activator. Accordingly, ways for predicting responsiveness to an α7-nAChR activator in patients with cognitive impairments or dysfunctions are urgently needed in the art.

Anatoly A. Mazurov et al (2011) Journal of Medicinal Chemistry, Vol. 54, No. 23, 7943-7961 discloses the discovery and development of α7 nicotinic acetylcholine receptor modulators.

Horenstein Nicole A. et al (2008) Molecular Pharmacology, Vol. 74, No 6, 1496-1511 discloses multiple pharmacophores for the selective activation of nicotinic α7-Type acetylcholine receptors.

### SUMMARY OF THE INVENTION

The present invention is defined with reference to the appended claims.

There is a need to predict whether a patient is likely to be responsive to treatment with an α7-nAChR activator. This objective is achieved by the methods and compositions provided within this disclosure. Disclosed are genetic variants of the chromosomal locus 15q24 which have been surprisingly shown to be predictive markers for responsiveness of patients suffering from cognitive impairments or dysfunctions to α7-nAChR activator treatment.

A first subject matter of the disclosure therefore relates to a composition comprising an alpha 7 nicotinic acetylcholine receptor activator for treatment of cognitive impairments, psychotic and/or neurodegenerative disorders in a selected patient population, wherein the patient population is selected on the basis of having at least one indicative SNP of the human acetylcholine receptor subunit alpha-5 (*CHRNA5*) gene (SEQ ID NO. 39; chromosome 15 NC_000015.9, cytogenetic location: 15q24; genomic coordinates (GRCh37): 78,857,861 - 78,887,610) or the human acetylcholine receptor subunit alpha-*3 (CHRNA3)* gene (SEQ ID NO. 40; chromosome 15, NC_000015.9, cytogenetic location: 15q24; genomic coordinates: 78885394..78913637, complement).

An indicative SNP, like rs55853698-T (SEQ ID NO. 1) as disclosed herein, is differentially present in human individuals and the likelihood of responsiveness of a patient can be predicted in the basis of the presence of the SNP rs55853698-T variant in the genome of said patient. Hence, an "indicative SNP" in the context of this application refers to a specific SNP that allows predicting in advance of treatment whether a patient suffering from cognitive impairments, psychotic and/or neurodegenerative disorders is likely to be responsive to treatment with an α7-nAChR activator. An indicative SNP in the context of this disclosure refers to a SNP present in the *CHNRA5* or *CHRNA3* gene and those forming a haplotype or in the same linkage disequilibrium with said SNPs. In another embodiment, the disclosure relates to a method for the identification of indicative SNPs of the *CHRNA5 or CHRNA3* genes comprising the steps of:
a) selecting a group of schizophrenic patients large enough to obtain statistically significant results; and
b) obtaining the genotype of said patients at the genetic locus of the *CHRNA5* or the *CHRNA3* gene; and
c) administering to said patients a therapeutic effective amount of an alpha 7 nicotinic acetylcholine receptor activator; and
d) performing an cognition assessment test with the patient of step c) by using a schizophrenic cognitive test battery (e.g. CogState™ schizophrenia Battery); and
e) subdivide the patients of step d) in responder and non-responder subgroups by identifying those patients showing statistically relevant improved visual learning, memory capabilities, improved cognitive function, improved reasoning, problem solving capabilities or improvements on attention and vigilance ("responders"), whereas the improvements, measured as effect size as described in the Example section below, are at least about 0.1, or about 0.2, or about 0.3, or about 0.4 or above about 0.5; and
f) analyse the DNA sequence of the genetic loci of the responder and non-responder subpopulations identified in step e) and select those SNPs only present at the genetic loci of the *CHRNA5* or *CHRNA3* gene of the responders;
g) identify heterozygous or homozygous indicative SNP variants by correlating the existence of the SNPs selected in step f) with the results of the cognition test of step d).

Methods for obtaining and analyzing the genotype of a patient at a defined locus or gene as well as the cognition assessment test are well known in the art and described in detail herein.

Preferably, the mentioned composition is used to treat cognitive impairments, psychotic and/or neurodegenerative disorders in which alpha 7 nicotinic acetylcholine receptor activation plays a role or is implicated. In one embodiment the cognitive impairments, psychotic and/or neurodegenerative disorders are selected from the group consisting of mild cognitive impairment, Alzheimer's disease, Parkinson's disease dementia, dementia with Lewy Bodies, schizophrenia, vascular dementia, AIDS-dementia, senile dementia, mild cognitive impairment related to age (MCI), age associated memory impairment, autism, dementias in frontal lobe degenerations, stroke, basalganglia degenerative disorders, multiple sclerosis, trauma, brain tumors, brain infections, hydrocephalus, depression, toxic or metabolic disorders and drug induced dementias.

In another embodiment the composition is used in patient population selected on the basis of carrying the human *CHRNA5* SNP rs55853698-T (SEQ ID No.1) or a SNP forming a haplotype together with said SNPs.

In yet another embodiment the SNP haplotype comprises at least two of the SNPs, rs3841324 (SEQ IDs NO.5 or 6), rs503464 (SEQ IDs NO.9 or 10), rs55853698-T (SEQ ID NO.1), rs55781567-C (SEQ ID NO. 7), rs56182392 (SEQ IDs NO. 11 or 12), rs77293642 (SEQ IDs NO. 13 or 14), rs67624739 (SEQ IDs NO. 15 or 16), rs142774214 (SEQ IDs NO. 17 or 18), rs60182379 (SEQ IDs NO. 19 or 20), rs77541452 (SEQ IDs NO. 21 or 22), rs72648882 (SEQ IDs NO. 23 or 24), rs144334096 (SEQ IDs NO. 25 or 26), rs114037126 (SEQ IDs NO. 27 or 28), rs140280786 (SEQ IDs NO. 29 or 30), rs147565924 (SEQ IDs NO. 31 or 32), rs16969968-G (SEQ ID NO. 37), rs6495308 (SEQ IDs NO.3 or 4), rs1051730 (SEQ IDs NO.35 or 36) and rs115662711 (SEQ IDs NO. 33 or 34) .

In certain embodiments, the composition for treatment of cognitive impairments, psychotic and/or neurodegenerative disorders is used in a patient population selected on the basis of carrying a SNP haplotype comprises the SNPs rs3841324 (SEQ ID NO.5 or 6), rs503464 (SEQ IDs NO.9 or 10), rs55853698-T (SEQ ID NO. 1) and rs55781567-C (SEQ ID NO.7) and wherein said SNPs forming the haplotype delTTC or insATC. In another embodiment, the composition for treatment of cognitive impairments, psychotic and/or neurodegenerative disorders is used in patients selected on the basis of being homozygous for the above mentioned indicative *CHRNA5* SNPs or the indicative *CHRNA5* SNP haplotypes, particularly being homozygous for the SNPs rs55853698-T/T (SEQ ID NO.1). Additionally, the disclosure relates to compositions for treatment of cognitive impairments, psychotic and/or neurodegenerative disorders, wherein the patient population is selected on the basis of carrying the indicative *CHRNA3* SNPs rs6495308 (SEQ ID NO.3 or 4), rs1051730 (SEQ IDs NO. 35 or 36) or SNPs forming a haplotype with said SNP's, wherein the homozygous presence of the SNP rs1051730-C/C (SEQ ID NO. 35) is an indication that the individual will likely respond to the alpha 7 nicotinic acetylcholine receptor activator treatment, and wherein the homozygous presence of the SNP rs6495308-T/T (SEQ ID NO. 4) is an indication that the individual will likely not respond to the alpha 7 nicotinic acetylcholine receptor activator treatment, whereas homozygous presence of the SNP rs6495308-C/C (SEQ ID NO. 3) or heterozygous presence of a SNP rs6495308-C/T (SEQ ID NO. 3/4) genotype is an indication that the individual will likely respond to the alpha 7 nicotinic acetylcholine receptor activator treatment.

Furthermore, the disclosure relates to compositions as described herein, and used for the methods, comprising an alpha 7 nicotinic acetylcholine receptor activator of formula (I) 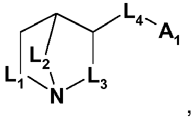
wherein L₁ is -CH₂-; L₂ is -CH₂- or -CH₂-CH₂-; and L₃ is -CH₂- or -CH(CH₃)-; or
L₁ is -CH₂-CH₂-; L₂ is -CH₂-; and L₃ is -CH₂-CH₂-;
L₄ is a group selected from 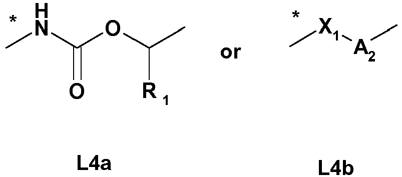
wherein the bond marked with the asterisk is attached to the azabicycloalkyl moiety; R₁ is hydrogen or C₁₋₄alkyl; X₁ is -O- or -NH-; A₂ is selected from 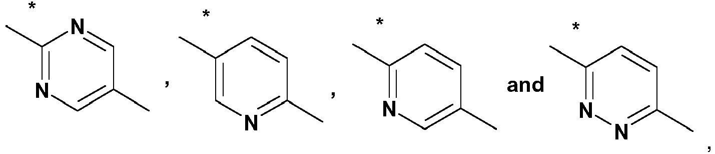
wherein the bond marked with the asterisk is attached to X₁; A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; each R₂ independently is C₁₋₆alkyl, C₁₋₆halogenalkyl, C₁₋₆alkoxy, C₁₋₆ halogenalkoxy, halogen, cyano or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C₁₋₆halogenalkoxy, halogen or cyano, and wherein a substituent on a nitrogen in a heterocyclic ring system may not be halogen; or two R₂ at adjacent ring atoms form a C₃₋₄alkylene group, wherein 1-2 carbon atoms may be replaced by X₂, and wherein the C₃₋₄alkylene group may be substituted once or more than once by R₃; each X₂ independently is -O- or -N(R₄)-; each R₄ independently is hydrogen or C₁₋₆alkyl; and each R₃ independently is halogen or C₁₋₆alkyl; in free base form or in acid addition salt form.

In the present invention the alpha 7 nicotinic acetylcholine receptor agonist is (R)-3-(6-p-tolyl-pyridin-3-yloxy)-1-aza-bicyclo[2.2.2]octane in free base form or in acid addition salt form.

An additional subject matter of the disclosure relates to a composition as described herein, and used for the methods, wherein the alpha 7 nicotinic acetylcholine receptor activator is used as free base or pharmaceutically acceptable acid addition salt form. In another embodiment, the alpha 7 nicotinic acetylcholine receptor activator is in its free base or pharmaceutically acceptable acid addition salt form, in association with a pharmaceutical carrier or a diluent.

In another embodiment of the disclosure, the composition as described herein, and used for the methods, further comprises a second cognition enhancer or a therapeutic compound useful for the treatment of cognitive impairments, psychotic and/or neurodegenerative disorders.

In yet another embodiment, the disclosure relates to a method for predicting therapeutic responsiveness of an individual or a group of individuals to alpha 7 nicotinic acetylcholine receptor activator treatment for increasing the cognitive skills and/or treatment of a cognitive impairment, psychotic and/or neurodegenerative disorder comprising the steps of: I) obtaining the genotype of the individual at the genetic locus of the *CHRNA5* gene; II) identifying those individuals of step I) carrying the *CHRNA5* SNP rs55853698-T (SEQ ID NO. 1) or a SNP forming a haplotype with said SNPs, wherein the presence of at least one of the SNPs or SNP haplotypes mentioned) above is an indication that the individual will likely respond to the alpha 7 nicotinic acetylcholine receptor activator treatment.

In yet another embodiment, the disclosure relates to a method for predicting therapeutic responsiveness of an individual or a group of individuals to alpha 7 nicotinic acetylcholine receptor activator treatment for increasing the cognitive skills and/or treatment of a cognitive impairment, psychotic and/or neurodegenerative disorder comprising the steps of: I) obtaining the genotype of the individual at the genetic locus of the *CHRNA3* gene; II) identifying those individuals of step I) carrying the *CHRNA3* SNP rs6495308 (SEQ ID NO.3 or 4) or the SNP rs1051730 (SEQ ID NO. 35) or a SNP forming a haplotype with said SNPs, wherein the homozygous presence of the SNP rs1051730-C/C (SEQ ID NO. 35) or SNP haplotypes mentioned above is an indication that the individual will likely respond to the alpha 7 nicotinic acetylcholine receptor activator treatment, and wherein the homozygous presence of the SNP rs6495308-T/T (SEQ ID NO. 4) is an indication that the individual will likely not respond to the alpha 7 nicotinic acetylcholine receptor activator treatment.

Homozygous presence of the SNP rs6495308-C/C (SEQ ID NO. 3) or heterozygous presence of a SNP rs6495308-C/T genotype is an indication that the individual will likely respond to the alpha 7 nicotinic acetylcholine receptor activator treatment.

Furthermore, the disclosure relates to a therapeutic method of increasing the cognitive skills of an individual and/or treatment of individuals suffering from a cognitive impairment, psychotic and/or neurodegenerative disorder comprising the steps of:
III) obtaining the genotype of the individual at the genetic locus of the *CHRNA5* gene;
IV) identifying those individuals of step III) carrying the *CHRNA5* SNP rs55853698-T (SEQ ID NO. 1) or a SNP forming a haplotype with said SNPs, wherein the presence of at least one of the SNPs or SNP haplotypes is an indication that the individual will likely respond to the alpha 7 nicotinic acetylcholine receptor activator treatment; or alternatively
III) obtaining the genotype of the individual at the genetic locus of the *CHRNA3* gene;
IV) identifying those individuals of step III) carrying the *CHRNA3* SNP rs6495308 (SEQ ID NO. 3 or 4) or the SNP rs1051730 (SEQ ID NO. 35) or a SNP forming a haplotype with said SNPs or a SNP in the same linkage disequilibrium with said SNPs, wherein the homozygous presence of the SNP rs1051730-C/C (SEQ ID NO. 35) is an indication that the individual will likely respond to the alpha 7 nicotinic acetylcholine receptor activator treatment, and wherein the homozygous presence of the SNP rs6495308-T/T (SEQ ID NO. 4) is an indication that the individual will likely not respond to the alpha 7 nicotinic acetylcholine receptor activator treatment. Homozygous presence of the SNP rs6495308-C/C (SEQ ID NO. 3) or heterozygous presence of a SNP rs6495308-C/T genotype is an indication that the individual will likely respond to the alpha 7 nicotinic acetylcholine receptor activator treatment, and
V) administering a therapeutic effective amount of an alpha 7 nicotinic acetylcholine receptor activator to those subject identified in step IV). In an additional embodiment, the above described steps I) and III) further comprising the steps of: VI) obtaining a biological sample of said individual, wherein said sample is selected from the group consisting of blood, blood-derived product (such as buffy coat, serum, and plasma), lymph, urine, tear, saliva, cerebrospinal fluid, buccal swabs, sputum, hair roots, leukocyte sample or tissue samples or any combination thereof, and VII) contacting the biological sample of step VI. with a reagent capable of detecting the (i) *CHRNA5* SNP rs55853698-T (SEQ ID NO.1), or the *CHRNA3* SNP rs6495308-C or -T (SEQ ID NO.3 or 4), or (iv) the SNP rs1051730-T or -C (SEQ ID NO. 35 or 36), or (v) a SNP forming a haplotype with said SNPs.

Preferably, the above mentioned methods are used to treat cognitive impairments, psychotic and/or neurodegenerative disorders in which alpha 7 nicotinic acetylcholine receptor activation plays a role or is implicated. Hence, in one embodiment, the above mentioned methods further comprising as a first step diagnosing the need for increasing the cognitive skills, or a cognitive impairment, psychotic and/or neurodegenerative disorder in an individual, whereas the cognitive impairments, psychotic and/or neurodegenerative disorders can be selected from the group consisting of mild cognitive impairment, Alzheimer's disease, Parkinson's disease dementia, dementia with Lewy Bodies, schizophrenia, vascular dementia, AIDS-dementia, senile dementia, mild cognitive impairment related to age (MCI), age associated memory impairment, autism, dementias in frontal lobe degenerations, stroke, basal ganglia degenerative disorders, multiple sclerosis, trauma, brain tumors, brain infections, hydrocephalus, depression, toxic or metabolic disorders and drug induced dementias.

In another embodiment of the disclosure the above mentioned methods, the presence of the *CHRNA5* SNP rs55853698-T (SEQ ID NO.1), or the *CHRNA3* SNP rs6495308-C or-T (SEQ ID NO.3 or 4), or the SNP rs1051730-T or -C (SEQ ID NO. 35 or 36) or a SNP forming a haplotype with said SNPs is determined by using at least one oligonucleotide that specifically hybridizes with specific regions on the nucleic acid molecule carrying said SNP or SNPs. Particularly, the presence of said *CHRNA5*/*CHRNA3* SNPs can be detected by sequence-specific primer (SSP) typing, sequence-specific oligonucleotide (SSO) typing, sequence based typing (SBT), DNA amplification such as polymerase chain reaction (PCR), microarray analysis, northern blot analysis, or reverse transcription PCR.

In another aspect of the disclosure, the individuals being selected according to the above describe methods as responders to alpha 7 nicotinic acetylcholine receptor activator treatment for increasing the cognitive skills and/or treatment of a cognitive impairment, psychotic and/or neurodegenerative disorder, are treated with a compound of formula (I). In yet another embodiment, a second cognition enhancer or a therapeutic compound useful for the treatment of cognitive impairments, psychotic and/or neurodegenerative disorders, such as a conventional antipsychotic or an atypical antipsychotic, can be co-administered.

In another aspect of the disclosed methods the alpha 7 nicotinic acetylcholine receptor activator dose to be administered is from about 2 mg to about 100 mg per day.

Another embodiment of the disclosure relates to the use of an alpha 7 nicotinic acetylcholine receptor activator for the treatment of a patient with cognitive impairments, psychotic and/or neurodegenerative disorders or condition in which alpha 7 nicotinic acetylcholine receptor activation plays a role or is implicated, wherein the patient being responsive to the treatment with an alpha 7 nicotinic acetylcholine receptor activator has been selected according to the above described methods.

Furthermore, the disclosure relates to the use of at least one probe for detecting the *CHRNA5* SNP rs55853698-T (SEQ ID NO.1), or the *CHRNA3* SNP rs6495308-C or -T (SEQ ID NO.3 or 4), or the SNP rs1051730-T or -C (SEQ ID NO. 35 or 36) or a SNP forming a haplotype with said SNPs for determining whether an individual is responsive to alpha 7 nicotinic acetylcholine receptor agonist treatment of cognitive impairments, psychotic and/or neurodegenerative disorders or condition in which alpha 7 nicotinic acetylcholine receptor activation plays a role or is implicated. In another aspect, the disclosure relates to a kit comprising at least one probe for detecting the *HRNA5* SNP rs55853698-T (SEQ ID NO.1), or the *CHRNA3* SNP rs6495308-C or -T (SEQ ID NO.3 or 4), or (iv) the SNP rs1051730-T or -C (SEQ ID NO. 35 or 36) or a SNP forming a haplotype with said SNPS. Preferably, said kit comprises VIII) means for detecting the (i) *CHRNA5* SNP rs55853698-T (SEQ ID NO.1), and/or (ii) the SNP rs16969968-G (SEQ ID NO. 37), and/or (iii) the *CHRNA3* SNP rs6495308-C or -T (SEQ ID NO.3 or 4), or (iv) the SNP rs1051730-T or -C (SEQ ID NO. 35 or 36) and/or (v) a SNP forming a haplotype with said SNPs or a SNP in the same linkage disequilibrium with said SNPs, and IX) instructions how to use said kit.

An additional subject matter of the disclosure relates to the use of a kit, preferably the above disclosed kit, suitable for any of the above described methods or uses, wherein said kit comprises at least one probe for detecting the *CHRNA5* SNP rs55853698-T (SEQ ID NO.1), or the *CHRNA3* SNP rs6495308-C or -T (SEQ ID NO.3 or 4), or the SNP rs1051730-T or -C (SEQ ID NO. 35 or 36) or a SNP forming a haplotype with said SNPs. In a related embodiment, the kit described above comprises oligonucleotide probes.

In another embodiment of the above described methods or uses the *CHRNA5*/*CHRNA3* SNP haplotype consists of at least two SNPs selected from the group consisting of rs3841324 (SEQ IDs NO.5 or 6), rs503464 (SEQ IDs NO.9 or 10), rs55853698-T (SEQ ID NO. 1), rs55781567-C (SEQ ID NO.7), rs56182392 (SEQ IDs NO. 11 or 12), rs77293642 (SEQ IDs NO. 13 or 14), rs67624739 (SEQ IDs NO. 15 or 16), rs142774214 (SEQ IDs NO. 17 or 18), rs60182379 (SEQ IDs NO. 19 or 20), rs77541452 (SEQ IDs NO. 21 or 22), rs72648882 (SEQ IDs NO. 23 or 24), rs144334096 (SEQ IDs NO. 25 or 26), rs114037126 (SEQ IDs NO. 27 or 28), rs140280786 (SEQ IDs NO. 29 or 30), rs147565924 (SEQ IDs NO. 31 or 32), rs16969968-G (SEQ ID NO. 37), rs6495308 (SEQ ID NO.4), rs1051730 (SEQ IDs NO. 35 or 36) and rs115662711 (SEQ IDs NO. 33 or 34). In another embodiment of the above described methods or uses the *CHRNA5* SNP haplotype consists of the indicative SNP rs55853698-T (SEQ ID NO. 1) and at least one additional SNP selected from the group consisting of rs3841324 (SEQ IDs NO.5 or 6), rs503464 (SEQ IDs NO.9 or 10), rs55781567-C (SEQ ID NO.7), rs56182392 (SEQ IDs NO. 11 or 12), rs77293642 (SEQ IDs NO. 13 or 14), rs67624739 (SEQ IDs NO. 15 or 16), rs142774214 (SEQ IDs NO. 17 or 18), rs60182379 (SEQ IDs NO. 19 or 20), rs77541452 (SEQ IDs NO. 21 or 22), rs72648882 (SEQ IDs NO. 23 or 24), rs144334096 (SEQ IDs NO. 25 or 26), rs114037126 (SEQ IDs NO. 27 or 28), rs140280786 (SEQ IDs NO. 29 or 30), rs147565924 (SEQ IDs NO. 31 or 32), rs16969968-G (SEQ ID NO. 37), rs6495308 (SEQ IDs NO.3 or 4), rs1051730 (SEQ IDs NO. 35 or 36) and rs115662711 (SEQ IDs NO. 33 or 34). In a related embodiment, the SNP haplotype is selected from the haplotype delTTC and insATC formed by the SNPs rs3841324 (SEQ ID NO.5 or 6), rs503464 (SEQ IDs NO.9 or 10), rs55853698-T (SEQ ID NO.1) and rs55781567-C (SEQ ID NO. 7).

### GENERAL DEFINITIONS

In order that the present disclosure may be more readily understood, certain terms are first defined. Additional definitions are set forth throughout the detailed description.

The term "comprising" means "including" *e.g.* a composition "comprising" X may consist exclusively of X or may include something additional *e.g.* X + Y.

The term "about" in relation to a numerical value x means, for example, *x*±10%.

The term "cognition enhancer" in the context of this disclosure refers to any drug, supplement, nutraceutical, or functional food that is said to improve mental functions such as cognition, memory, intelligence, motivation, attention, and concentration. Cognition enhancers, as used herein include, but are not limited to cholinergic compounds like acetylcholine esterase inhibitors and/or buturylesterase inhibitors (rivastigmine, donezepil, galantamine, huperzine), ampakines (e.g. CX614, CX516), muscarinic modulators (e.g. muscarinic receptor agonists), modulators of the NMDA-receptor (e.g. positive modulators, antagonists, memantine), phosphodiesterase inhibitors (e.g. PDE4 inhibitors), nootropic compounds like hydergine, oxiracetam, aniracetam, acetyl-L-carnitine, ginko-derived compounds, compounds contained in gerovitals like p-aminobenzoic acid and dithylaminoethanol and derivative thereof and attention-modulating compounds like methylphenicate, tomoxetine and modafinil.

The term "conventional antipsychotics" denotes compounds that are effective in treating psychoses mainly via dopamine receptor D2 antagonism. "Conventional antipsychotics" as used herein includes, but is not limited to haloperidol, droperidol, molindone, fluphenazine, thiotixene, flupentixol, promazine, pimozide, chlorpromazine, methotrimeprazine, pipotiazine trifluoperazine, thioridazine, acetophenazine, chlorprothixene and mesoridazine.

The term "atypical antipsychotics" denotes compounds that are effective in treating psychoses via an additional and/or different mechanism than dopamine receptor 2 antagonism. "Atypical antipsychotics" as used herein includes, but is not limited to clozaril, risperidone, olanzapine, quetiapine, ziprasidone, aripiprazol, sertindole, perphenazine, mesoridazine, prochlorperazine, naproxene and loxapine.

As used herein, the term alpha 7 nicotinic acetylcholine receptor activator (α7-nAChR activators) refers to α7-nAChR agonists and α7-nAChR positive allosteric modulators, particularly to low molecular weight (LMW) compounds as disclosed herein.

The term "SNP" in the context of this disclosure refers to a "single nucleotide polymorphism". A "SNP" is a genetic variation between individuals; e.g., a single base position in the DNA of organisms that is variable. A SNP defines a specific allele of a given gene. As used herein, "SNPs" is the plural of SNP. A SNP occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). SNPs are most frequently diallelic. A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference. A SNP may also refer to a polymorphic site on a single chromosome or within a region of a single chromosome, wherein the SNP might refer to an insertion or deletion of several base pairs (e.g. the herein disclosed SNP rs3841325 refers to an insertion/deletion of 22 base pairs at postion -71 upstream of the transcription start site of the human *CHRNA5* gene). Hence, the term "SNP" refers also to a region of a gene having one of several nucleotide sequences found in that region of the gene in different individuals in a population. Although most SNPs are rare, it has been estimated that there are 5.3 million common SNPs, each with a frequency of 10-50%, that account for the bulk of the DNA sequence difference between humans. Such SNPs are present in the human genome once every 600 base pairs (Kruglyak and Nickerson, Nature Genet. 27:235 (2001)). Alleles (variants) making up blocks of such SNPs in close physical proximity are often correlated, resulting in reduced genetic variability and defining a limited number of "SNP haplotypes" (Fullerton, et al., Am. J. Hum. Genet. 67:881 (2000)).

The term "gene" refers to a coding region operably linked to appropriate regulatory sequences capable of regulating the expression of the polypeptide in some manner. A gene includes untranslated regulatory regions of DNA (e.g., promoters, enhancers, repressors, etc.) preceding (upstream) and following (downstream) the coding region (open reading frame, ORF) as well as, where applicable, intervening sequences (i.e., introns) between individual coding regions (i.e., exons). Genes may also include sequences located on both the 5'- and 3'-end of the sequences, which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers, which control or influence the transcription of the gene. The 3'-flanking region may contain sequences, which direct the termination of transcription, posttranscriptional cleavage and polyadenylation.

The term "mild cognitive impairment" (MCI) in the context of this disclosure refers to a cognitive impairment beyond that expected for an individual of a certain age and education, but that do not interfere significantly with the daily activities of such an individual (Petersen RC, Smith GE, Waring SC, Ivnik RJ, Tangalos EG, Kokmen E (1999). "Mild cognitive impairment: clinical characterization and outcome". Arch. Neurol. 56 (3): 303-8.)

The term "haplotype" in the context of this disclosure refers to a group of SNPs that do not appear to recombine independently and that can be grouped together in blocks of SNPs. Hence, SNPs that constitute a haplotype are in linkage disequilibrium and thus tend to be inherited together. In a preferred embodiment, a SNP haplotype refers to a combination of SNPs present in the human *CHRNA5* gene (SEQ ID NO. 39) and/or the *CHRNA3* gene (SEQ ID NO. 40), wherein the SNPs forming said haplotype are located in an area of about 100000, or about 50000, or about 30000, or about 20000, or about 10000 base-pairs upstream and/or downstream of the position of a given SNP in the genome. "Haplotype" also refers to the particular combinations of polymorphic variants (SNPs and/or alleles) observed in a population at polymorphic sites on a single chromosome or within a region of a single chromosome. A "haplotype," as described herein, refers to any combination of SNPs or polymorphic sites. A haplotype can comprise two or more SNPs/alleles and the length of a genome region comprising a haplotype may vary from few hundred bases up to hundreds of kilo bases. It is recognized by those skilled in the art that the same haplotype can be described differently by determining the haplotype defining alleles from different nucleic acid strands. E.g. the haplotype delTTC defined by the SNPs rs3841324-Del (SEQ ID NO. 6), rs503464-T (SEQ ID NO. 10), rs55853698-T (SEQ ID NO. 1) and rs55781567-C (SEQ ID NO.7) of this ddisclosure is the same haplotype as GAAdel defined by the same SNPs in which the alleles are determined from the other strand, or delATC in which the second SNP is determined from the other strand. SNPs described herein are differentially present in human individuals and their specific sequence is indicative for the responsiveness to alpha 7 nicotinic acetylcholine receptor activator treatment. Therefore, these SNPs and the haplotypes comprising said SNPs have diagnostic value for risk assessment and treatment efficacy in an individual. Detection of SNPs or polymorphic regions forming haplotypes can be accomplished by methods known in the art used for detecting nucleotides at polymorphic sites (see also definition of Linkage disequilibrium below).

"Linkage disequilibrium" or "LD" refers to a situation, in which two or more allelic variants are linked, i.e., there is a non-random correlation between allelic variants at two or more polymorphic sites in individuals in a population. LD is commonly denoted by a capital D. Normalizing D by dividing it by the theoretical maximum for the observed allele frequencies results in D'. A value of 0 for D' indicates that the examined loci are in fact independent of one another, while a value of 1 demonstrates complete dependency. Two or more allelic variants/SNPs that are linked are said to be in linkage disequilibrium. In general, allelic variants that are part of a haplotype or haplotype block are in linkage disequilibrium. A variety of methods/metrics are known in the art to evaluate the extent to which any two polymorphic variants (alleles) or SNPs are in LD. Suitable metrics include D', r2, and others (see, e.g., Hedrick, P. W., Genetics, 117(2):331-41, 1987). As used herein, polymorphic variants or SNPs are in "strong LD", and forming a haplotype if D'>0.8.

The term "subject" as used herein refers preferably to a human being, especially to a patient being diagnosed with a cognitive impairment, schizophrenia or an other mental disease which is a acquired deficit in one or more of memory function, problem solving, orientation and/or abstraction that impinges on an individual's ability to function independently. Subject, patient or individual are used interchangeably.

The term "cognitive disorders/impairments" and "psychotic and/or neurodegenerative disorders" refer to a mental diseases which are acquired deficits in one or more of memory function, problem solving, orientation and/or abstraction that impinges on an individual's ability to function independently. Examples of said disorders are Alzheimer's disease, Lewy Body Dementia, Amyotrophic Lateral Sclerosis, memory impairment, memory loss, cognition deficit, attention deficit, hyperactivity Disorder, schizophrenia, Parkinson's disease dementia and vascular dementia.

### DETAILED DESCRIPTION OF THE INVENTION

It has been discovered that genetic variants present on the human acetylcholine receptor subunit alpha-5 (*CHRNA5*) gene (SEQ ID NO. 39) or the human neuronal acetylcholine receptor subunit alpha-3 (*CHRNA3*) gene (SEQ ID NO. 40) are markers for predicting therapeutic responsiveness to an α7-nAChR activator therapy in a subject suffering from cognitive impairments or dysfunctions, psychotic and/or neurodegenerative disorders. Nicotinic acetylcholine receptors (nAChRs), such as *CHRNA5 or CHRNA3,* are members of a superfamily of ligand-gated ion channels that mediate fast signal transmission at synapses. The herein disclosed teaching provides a method for treating cognitive impairments or dysfunctions, psychotic and/or neurodegenerative disorders with α7-nAChR activators, based upon the presence of certain indicative SNPs in the human *CHRNA5* and/or *CHRNA3* gene.

The methods, compositions and kits of the present disclosure therefore provide a means for selecting patients suffering from cognitive impairments or dysfunctions, psychotic and/or neurodegenerative disorders that are more likely to respond α7-nAChR activator therapy, thereby enhancing the therapeutic efficacy of such treatments.

Therefore, in one aspect, the disclosureprovides a composition comprising an alpha 7 nicotinic acetylcholine receptor activator for the treatment of cognitive impairments, psychotic and/or neurodegenerative disorders in a selected patient population, wherein the patient population is selected on the basis of the genotype of the patients at the human acetylcholine receptor subunit alpha-5 (*CHRNA5*) gene or the human neuronal acetylcholine receptor subunit alpha-3 (*CHRNA3*) gene and wherein said genotype is indicative of efficacy of alpha 7 nicotinic acetylcholine receptor activator treatment. The specific marker for predicting therapeutic responsiveness to an α7-nAChR activator therapy can be a SNP and/or a polymorphic region. The skilled person is aware of the nucleic acid sequence and the location of the *CHRNA5* gene (located on the human chromosome: 15 (NC_000015.9 (78857862..78887611) and the *CHRNA3* gene (located on the human chromosome: 15 (NC_000015.9 (78885394..78913637, complement).

In order that the present disclosure may be more readily understood, the SNPs disclosed herein are defined as follows:

| SNP name and SEQ ID | Sequence |
|---|---|
| rs55853698 (SEQ ID NO. 1 or 2) | CGGGAGCTGTGGCGCGGAGCGGCCCC[G/T]CTGCTGCGTCTGCCCTCGT TTTGTC |
| rs55853698-T (SEQ ID NO. 1) | |
| rs55853698-G (SEQ ID NO. 2) | |
| rs6495308 (SEQ ID NO. 3 or 4) | GTAACTGTCTGATGGCAGGTTTGCTG[C/T]TGGGAGAGTAGAGAAGAGGT TTGGG |
| rs6495308-C (SEQ ID NO. 3) | |
| rs6495308-T (SEQ ID NO. 4) | |
| rs3841324 (SEQ ID NO. 5 or 6) | GAAGCTCCCGGCGCCTAGCCCCGCCC[- /CTATTTCCCTCTGGCCCCGCCC]AATCCTCGCCTTGTTCCTTTTGGCC |
| rs3841324-Ins (SEQ ID NO. 5) | |
| rs3841324-Del (SEQ ID NO. 6) | |
| rs55781567 (SEQ ID NO. 7 or 8) | TTGTCTCACGACTCACACTCAGTGCT[C/G]CATTCCCCAAGAGTTCGCGTT CCCC |
| rs55781567-C (SEQ ID NO. 7) | |
| rs55781567-G (SEQ ID NO. 8) | |
| rs503464 (SEQ ID NO. 9 or 10) | ACAGCTCCCGCCGGGACAGCAGCCTC[A/T]GCCTAGCAGCTTCTGGCGG GCTCGG |
| rs503464-A (SEQ ID NO. 9) | |
| rs503464-T (SEQ ID NO. 10) | |
| rs56182392 (SEQ ID NO. 11 or 12) | GGAGCTTCCACATGCGTCCCGAGCCC[A/G]CCAGAAGCTGCTAGGCTGA GGCTGC |
| rs56182392-G (SEQ ID NO. 11) | |
| rs56182392-A (SEQ ID NO. 12) | |
| rs77293642 (SEQ ID NO. 13 or 14) | CTGCTAGGCTGAGGCTGCTGTCCCGG[C/T]GGGAGCTGTGGCGCGGAGC GGCCCC |
| rs77293642-C (SEQ ID NO. 13) | |
| rs77293642-T (SEQ ID NO. 14) | |
| rs67624739 (SEQ ID NO. 15 or 16) | GGAACAAGGCGAGGATTGGCGGGGC[- /CAGAGGGAAATAGGGGCGGGGC ]TAGGCGCCGGGAGCTTCCACATGCG |
| rs67624739-Ins (SEQ ID NO. 15) | |
| rs67624739-Del (SEQ ID NO. 16) | |
| rs142774214 (SEQ ID NO. 17 or 18) | GGGCCAAAAGGAACAAGGCGAGGATT[- /GGGCGGGGCCAGAGGGAAATAG ]GGGCGGGGCTAGGCGCCGGGAGCTT |
| rs142774214-Ins (SEQ ID NO. 17) | |
| rs142774214-Del (SEQ ID NO. 18) | |
| rs60182379 (SEQ ID NO. 19 or 20) | GGGGATCGGAGGCGGGGCTATGAATA[G/T]ACCGGACTGGGCCAAAAGG AACAAG |
| rs60182379-G (SEQ ID NO.19) | |
| rs60182379-T (SEQ ID NO. 20) | |
| rs77541452 (SEQ ID NO. 21 or 22) | GACAGGGTTGGACCAGAGGCGGGGCG[A/G]GGTGGGAAAGGGTTGGT GGGGATC |
| rs77541452-A (SEQ ID NO. 21) | |
| rs77541452-G (SEQ ID NO. 22) | |
| rs72648882 (SEQ ID NO. 23 or 24) | TGTTATCTTAAGGTGTGCAGTGACTA[G/T]GCCAAGGACATCCGGTGGTTA GAGA |
| rs72648882-G (SEQ ID NO. 23) | |
| rs72648882-T (SEQ ID NO. 24) | |
| rs144334096 (SEQ ID NO. 25 or 26) | AGAGCAGACATCAATGAATAGCTGTT[A/T]TCTTAAGGTGTGCAGTGACTA GGCC |
| rs144334096-A (SEQ ID NO. 25) | |
| rs144334096-T (SEQ ID NO. 26) | |
| rs114037126 (SEQ ID NO. 27 or 28) | AGCACAGAGCAGACATCAATGAATAG[C/T]TGTTATCTTAAGGTGTGCAGT GACT |
| rs114037126-C (SEQ ID NO. 27) | |
| rs114037126-T (SEQ ID NO. 28) | |
| rs140280786 (SEQ ID NO. 29 or 30) | CGGCTCCCTCTGCCACTGAAGTTTAA[A/G]GATAATTCGCCTGAATCCCTT CAGC |
| rs140280786-A (SEQ ID NO. 29) | |
| rs140280786-G (SEQ ID NO. 30) | |
| rs147565924 (SEQ ID NO. 31 or 32) | TTCTTTGCAACCAGGTTTAGTTTTTC[A/C]CAGATTTACTAGTCTGGAGGGT GGA |
| rs147565924-A (SEQ ID NO. 31) | |
| rs147565924-C (SEQ ID NO. 32) | |
| rs115662711 (SEQ ID NO. 33 or 34) | TGGCTCCATTGGATGTAATTTACATT[C/T]TTCCAGCTGTATTTGGACCTAA GAA |
| rs115662711-C (SEQ ID NO. 33) | |
| rs115662711-T (SEQ ID NO. 34) | |
| rs1051730 (SEQ ID NO. 35 or 36) | TGGGCCATCATCAAAGCCCCAGGCTA[C/T]AAACACGACATCAAGTACAAC TGCT |
| rs1051730-C (SEQ ID NO. 35) | |
| rs1051730-T (SEQ ID NO. 36) | |
| rs16969968 (SEQ ID NO. 37 or 38) | CTAGAAACACATTGGAAGCTGCGCTC[A/G]ATTCTATTCGCTACATTACAA GACA |
| rs16969968-G (SEQ ID NO. 37) | |
| rs16969968-A (SEQ ID NO. 38) | |

Preferably, the mentioned composition is used as described herein to treat cognitive impairments, psychotic and/or neurodegenerative disorders in which alpha 7 nicotinic acetylcholine receptor activation plays a role or is implicated by increasing the cognitive skills of an individual. In one embodiment the cognitive impairments, psychotic and/or neurodegenerative disorders are selected from the group consisting of mild cognitive impairment, Alzheimer's disease, Parkinson's disease dementia, dementia with Lewy Bodies, vascular dementia, AIDS-dementia, senile dementia, mild cognitive impairment related to age (MCI), age associated memory impairment, autism, dementias in frontal lobe degenerations, stroke, basal ganglia degenerative disorders, multiple sclerosis, trauma, brain tumors, brain infections, hydrocephalus, depression, toxic or metabolic disorders and drug induced dementias. In another embodiment of the disclosure the mentioned composition is used as described herein to treat patients suffering from schizpophrenia.

Treatment of the patient population selected based upon the presence of certain indicative SNPs in the human *CHRNA5* and/or *CHRNA3* gene with α7-nAChR activators leads to statistically relevant improved visual learning and memory capabilities, improved cognitive function, improved reasoning and problem solving capabilities and improvements on attention and vigilance compared to individuals not carrying the indicative SNPs, whereas the improvements, measured as effect size as described in the Example section, are at least 0.1, or 0.2, or 0.3, or 0.4 or above 0.5. The effect size values will differ depending on the applied tests and treatment condition.

The phrase "increasing the cognitive skills of an individual" refers to a situation in which (i) the cognitive skills or capabilities, the physiological condition and/or the psychological condition of an individual would be considered by a skilled person as being outside of the normal range of a healthy individual, and (ii) wherein treatment with the compositions of the disclosure or according to the method leads to a significant improvement compared to individuals of a control group (e.g. individuals who do not carry an indicative marker SNP) or placebo group. The improvement can be complete (e.g. the skilled person would consider the patient as being inside the normal range) or partial, such that the peculiarity of the above mentioned condition in a subject is significantly less pronounced than had the subject not received a composition or treatment according to the present disclosure. Partial treatment results may lead to a decrease in severity of the above mentioned conditions or disease symptoms, an increase in frequency and duration of disease symptom-free periods, or a prevention of impairment or disability due to the disease affliction. In this context, the term "significantly less pronounced" refers to a conditions or status of a person which, on the basis of measurement of relevant parameter, would after the treatment with the compositions of the disclosure or according to the method, not be considered by a skilled person as being completely healthy (parameters might be still outside the normal range), but where a significant improvement (which could be an increase or decrease of a certain parameter) of a relevant parameter has been observed. A significant improvement or decrease can be identified for example by comparison of the treatment results of individual patients compared to individuals of a control group (e.g. individuals who do not carry an indicative marker SNP) or placebo group. The skilled person is well aware of the relevant parameter for cognitive skills or capabilities, physiological condition and/or psychological condition of an individual and how to determine them. Said parameters might be selected by a skilled person (e.g. a physician) on the basis of the investigated age-related condition. The phrase "increasing the cognitive skills of an individual" refers also to a situation in which an individual that would be considered (regarding cognitive skills or capabilities) by a skilled person as being inside of the normal range of a healthy individual wants to increase its cognitive skills or capabilities.

In one embodiment, the composition is used in patient populations selected on the basis of carrying the human *CHRNA5* SNP rs55853698-T as disclosed in SEQ ID NO.1. The SNP rs55853698 is a G/T allele (SEQ IDs NO. 1 and 2) with a base pair length of 1 located at position 78,857,939 of the human chromosome 15.

The skilled person is aware of the fact that several SNPs or polymorphic regions exist in the human *CHRNA5* gene or in the genomic regions of the human chromosome 15 forming a haplotype with the SNP rs55853698-T (SEQ ID NO.1). Said SNPs or polymorphic regions might be located in an area of about 100000, or about 50000, or about 30000, or about 20000, or about 10000 basepairs upstream and downstream of the position of the SNP rs55853698-T (SEQ ID NO.1). SNPs or polymorphic regions forming a haplotype with the SNP rs55853698-T (SEQ ID NO.1) will be equally suited to be used as markers for predicting therapeutic responsiveness to an α7-nAChR activator. The skilled person is aware of methods to identify other SNPs or polymorphic regions forming a haplotype with the SNP rs55853698-T (SEQ ID NO.1) ((see, e.g., Hedrick, P. W., Genetics, 117(2):331-41, 1987 and definition section above). Therefore, in another aspect, the disclosure provides a composition comprising an alpha 7 nicotinic acetylcholine receptor activator for the treatment of patients suffering from cognitive impairments, psychotic and/or neurodegenerative disorders, aiming to increase the cognitive skills of aid patients, wherein the patient population is selected on the basis of the presence of a SNP or polymorphic region forming a haplotype with the SNP rs55853698-T (SEQ ID NO.1). In yet another embodiment the disclosure also provides a composition comprising an alpha 7 nicotinic acetylcholine receptor activator for the treatment of patients suffering from cognitive impairments, psychotic and/or neurodegenerative disorders, aiming to increase the cognitive skills of aid patients, wherein the patient population is selected on the basis of the presence of at least one of the following SNPs: rs3841324 (SEQ ID NO. 5 or 6), rs503464 (SEQ IDs NO.9 or 10), rs55781567-C (SEQ ID NO. 7), rs56182392 (SEQ ID NO. 11 or 12), rs77293642 (SEQ IDs NO. 13 or 14), rs67624739 (SEQ IDs NO. 15 or 16), rs142774214 (SEQ IDs NO. 17 or 18), rs60182379 (SEQ IDs NO. 19 or 20), rs77541452 (SEQ IDs NO. 21 or 22), rs72648882 (SEQ IDs NO. 23 or 24), rs144334096 (SEQ IDs NO. 25 or 26), rs114037126 (SEQ IDs NO. 27 or 28), rs140280786 (SEQ IDs NO. 29 or 30), rs147565924 (SEQ IDs NO. 31 or 32) and/or rs115662711 (SEQ IDs NO. 33 or 34) forming a haplotype with the SNP rs55853698-T (SEQ ID NO.1) and/or the SNP rs16969968-G (SEQ ID NO. 37). Based on the above, the composition comprising an alpha 7 nicotinic acetylcholine receptor activator for the treatment of cognitive impairments, psychotic and/or neurodegenerative disorders can be used in a patient population selected on the basis of carrying the SNPs rs3841324-del (SEQ ID NO 6), rs503464-T (SEQ ID NO. 10) and/or rs55781567-C (SEQ ID NO.7) because said SNPs forming the haplotype delTTC with rs55853698-T (SEQ ID NO. 1). Alternatively, the patient population can be selected on the basis of carrying the SNPs rs3841324-ins (SEQ ID NO. 5), or rs503464-A (SEQ IDs NO. 9) and rs55781567-C (SEQ ID NO. 7), because said SNPs forming the haplotype insATC with rs55853698-T (SEQ ID NO. 1).

In another aspect of the disclosure, the patient population can also be selected on the basis of the genotype of patients at the human neuronal acetylcholine receptor subunit alpha-3 (*CHRNA3*). In one embodiment, the composition is used for the treatment of patients suffering from cognitive impairments, psychotic and/or neurodegenerative disorders, aiming to increase the cognitive skills of said patients, in a patient population selected on the basis of carrying the human *CHRNA3* SNP rs6495308 as disclosed in SEQ ID NO. 3/4. The SNP rs6495308 is a C/T allele variant with a base pair length of 1 located at position 78,907,656 of the human chromosome 15 (SEQ IDs NO. 3 and 4). In yet another embodiment the disclosure also provides a composition comprising a alpha 7 nicotinic acetylcholine receptor activator for the treatment of patients suffering from cognitive impairments, psychotic and/or neurodegenerative disorders aiming to increase the cognitive skills of said patients, wherein the patient population is selected on the basis of the presence of the SNP rs1051730 (SEQ ID NO. 35). The SNP rs1051730 is a C/T allele variant with a base pair length of 1 located at position 78,894,339 of the human chromosome 15.

Several SNPs or polymorphic regions exist in the human *CHRNA3* gene (e.g. SNP rs6495308 (SEQ IDs NO.3 or 4) or SNP rs1051730 (SEQ IDs NO. 35 or 36). Said SNPs or polymorphic regions might be located in an area of about 100000, or about 50000, or about 30000, or about 20000, or about 10000 base-pairs upstream and downstream of the position of rs6495308 (SEQ ID NO. 3/4) or SNP rs1051730 (SEQ ID NO. 35/36). SNPs or polymorphic regions forming a haplotype with the SNP rs6495308 (SEQ ID NO. 3/4) or SNP rs1051730-C (SEQ ID NO. 35/36) will be equally suited to be used as markers for predicting therapeutic responsiveness to a α7-nAChR activator. Therefore, in another aspect, the disclosure provides a composition comprising a alpha 7 nicotinic acetylcholine receptor activator for the treatment of cognitive impairments, psychotic and/or neurodegenerative disorders in a selected patient population, wherein the patient population is selected on the basis of the presence of a SNP or polymorphic region forming a haplotype with the rs6495308 (SEQ ID NO. 3/4) or SNP rs1051730-C (SEQ ID NO. 35/36). The homozygous presence of the SNP rs6495308-T/T (SEQ ID NO. 4) is an indication that the individual will likely not respond to the alpha 7 nicotinic acetylcholine receptor activator treatment. Homozygous presence of the SNP rs6495308-C/C (SEQ ID NO. 3) or heterozygous presence of a SNP rs6495308-C/T genotype is an indication that the individual will likely respond to the alpha 7 nicotinic acetylcholine receptor activator treatment. The homozygous presence of the SNP rs1051730-C/C (SEQ ID NO. 35) is an indication that the individual will likely respond to the alpha 7 nicotinic acetylcholine receptor activator treatment.

In another embodiment of the disclsoure, the composition for treatment of patients suffering from cognitive impairments, psychotic and/or neurodegenerative disorders is used in patients selected on the basis of being homozygous/heterozygous for the above mentioned indicative *CHRNA5* or *CHRNA3* SNPs or the indicative *CHRNA5 or CHRNA3* SNP haplotypes, particularly being homozygous for the SNPs rs55853698-T/T (SEQ ID NO.1), rs1051730-C/C (SEQ ID NO. 35) and/or being homozygous/heterozygous for the SNP rs6495308-C/C or C/T (SEQ ID NO. 3/4) or SNPs forming a haplotype with said SNPs or a SNP in the same linkage disequilibrium with said SNPs.

### LMW α7-nAChR activators:

### LMW α7-nAChR agonists:

In one embodiment, the α7-nAChR activator used is an α7-nAChR agonist.

An α7-nAChR agonist is a compound that binds to a receptor comprising an α7-nAChR subunit in vivo and in vitro and is activating the receptor. Activation can be measured by the method disclosed in WO2001/85727, i.e. a functional affinity assay at the homomeric α 7-nAChR carried out with a rat pituitary cell line stably expressing the α7-nAChR. As read out, the calcium influx upon stimulation of the receptor compared to epibatidine is used. "α7-nAChR agonists" according to the disclosure typically induce calcium influx of at least 50% of the maximal influx evoked by epibatidine with an EC₅₀ value of at least 1µM; preferred agonists induce calcium influx of at least 75% of the maximal influx evoked by epibatidine with an EC₅₀ value of at least 400nM; more preferred agonists induce calcium influx of at least 85% of the maximal influx evoked by epibatidine with an EC₅₀ value of at least 50nM.

Preferred α7-nAChR agonists should be well absorbed from the gastrointestinal tract, should be sufficiently metabolically stable and possess favorable pharmacokinetic properties. Further preferred α7-nAChR agonists bind in-vivo potently to α7-nAChRs whilst showing little affinity for other receptors, especially for other nAChRs, e.g. α4β2 nAChR, for muscarinic acetylcholine receptors, e.g. M1, and/or the 5-HT₃ receptor. Further preferred α7-nAChR agonists cross the blood brain barrier effectively. Preferred α 7-nAChR agonists should be non-toxic and demonstrate few side-effects. Furthermore, a preferred α7-nAChR agonist will be able to exist in a physical form that is stable, non-hygroscopic and easily formulated.

In one embodiment, the α7-nAChR agonist is a selective α7-nAChR agonist, i.e. is selective for a receptor comprising an α7-nAChR subunit, since such an agonist would be expected to cause fewer side effects than a non-selective agonist to a treated subject. An agonist being selective for a receptor comprising an α7-nAChR subunit has a functional affinity to such a receptor to a much higher degree, e.g. at least 10-fold affinity difference in EC₅₀ value, preferably at least 20-fold, more preferably at least 50-fold, compared to any other nicotinic acetylcholine receptor. To assess the affinity of the α7-nAChR agonists of the disclosure on other nicotinic acetylcholine receptors, the method disclosed in WO2001/85727 can be used, i.e. to assess the affinity on human α4β2 nAChR, a similar functional assay is carried out using a human embryonic kidney cell line stable expressing the human α4β2 subtype and to assess the activity of the compounds of the disclosure on the "ganglionic subtype" and the "muscle type" of nicotinic receptor, similar functional assays are carried out with a human embryonic kidney cell line stably expressing the human "ganglionic subtype" or a cell line endogenously expressing the human "muscle type" of nicotinic receptors.

In the last 15 years much effort has been focused on developing selective α7 nAChR agonists leading to the discovery of many different chemotypes displaying said selective activity. These efforts are summarized the review from Horenstein et al (Mol Pharmacol, 2008, 74, 1496-1511, which describes no less than 9 different families of α7 nAChR agonists, in most of which selective agonists have been found. In fact, several drug candidates having an α7 nAChR agonist mode of action entered pre-clinical or even clinical testing (for review: Broad et al, Drugs of the Future, 2007, 32(2), 161-170; Romanelli et al, Expert Opin Ther Patents, 2007, 17(11), 1365-1377). Examples of such compounds - again belonging to a diversity of chemotypes - are MEM3454, MEM63908, SSR180711, GTS21, EVP6124, ABT107, ABT126, TC-5619, AZD-6319 and SAR-130479. Further α7 nAChR agonists and their use as pharmaceuticals are known, for example, from WO2001/85727, WO2004/022556, WO2005/123732, WO2006/005608, WO2007/045478, WO2007/068476 and WO2007/068475.

In one embodiment, the α7-nAChR agonist has a maximum molecular weight of 1500 daltons. In one embodiment, the α7-nAChR agonist has a maximum molecular weight of 1000 daltons. In one embodiment, the α7-nAChR agonist has a maximum molecular weight of 800 daltons. In one embodiment, the α7-nAChR agonist has a maximum molecular weight of 500 daltons.

In one embodiment, the α7-nAChR agonist is a compound of formula (I) 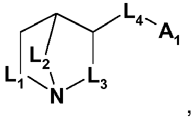 wherein
L₁ is -CH₂-; L₂ is -CH₂- or -CH₂-CH₂-; and L₃ is -CH₂- or -CH(CH₃)-; or
L₁ is -CH₂-CH₂-; L₂ is -CH₂-; and L₃ is -CH₂-CH₂-;
L₄ is a group selected from 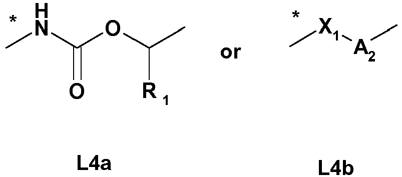
wherein the bond marked with the asterisk is attached to the azabicycloalkyl moiety;
R₁ is hydrogen or C₁₋₄alkyl;
X₁ is -O- or -NH-;
A₂ is selected from

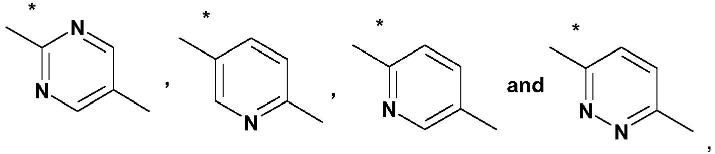

wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen;
each R₂ independently is C₁₋₆alkyl, C₁₋₆halogenalkyl, C₁₋₆alkoxy, C₁₋₆halogenalkoxy, halogen, cyano or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C_{1- 6}halogenalkyl, C₁₋₆alkoxy, C₁₋₆halogenalkoxy, halogen or cyano, and wherein a substituent on a nitrogen in a heterocyclic ring system may not be halogen;
or two R₂ at adjacent ring atoms form a C₃₋₄alkylene group, wherein 1-2 carbon atoms may be replaced by X₂, and wherein the C₃₋₄alkylene group may be substituted once or more than once by R₃;
each X₂ independently is -O- or -N(R₄)-;
each R₄ independently is hydrogen or C₁₋₆alkyl; and
each R₃ independently is halogen or C₁₋₆alkyl;
in free base form or in acid addition salt form.

Unless indicated otherwise, the expressions used in this disclosure have the following meaning:
"Alkyl" represents a straight-chain or branched-chain alkyl group, for example, methyl, ethyl, n- or iso-propyl, n-, iso-, sec- or tert-butyl, n-pentyl, n-hexyl; C₁₋₆alkyl preferably represents a straight-chain or branched-chain C₁₋₄alkyl with particular preference given to methyl, ethyl, n-propyl, iso-propyl and tert-butyl.

Each alkyl part of "alkoxy", "halogenalkyl" and so on shall have the same meaning as described in the above-mentioned definition of "alkyl", especially regarding linearity and preferential size.

A substituent being substituted "once or more than once", for example as defined for A₁, is preferably substituted by one to three substituents.

Halogen is generally fluorine, chlorine, bromine or iodine; preferably fluorine, chlorine or bromine. Halogenalkyl groups preferably have a chain length of 1 to 4 carbon atoms and are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,2-trichloroethyl, 1,1,2,2-tetrafluoroethyl, 2,2,3,3-tetrafluoropropyl, 2,2,3,3,3-pentafluoropropyl or 2,2,3,4,4,4-hexafluorobutyl; preferably -CF3, -CHF2, -CH2F, -CHF-CH3, -CF2CH3, or -CH2CF3.

In the context of the disclosure, the definitions of "two R2 at adjacent ring atoms form a C3-4alkylene group, wherein 1-2 carbon atoms may be replaced by X2" or "two R5 at adjacent ring atoms form a C3-4alkylene group, wherein 1-2 carbon atoms may be replaced by X3"
encompass -CH₂-CH₂-CH₂-, -CH₂-CH₂-CH₂-CH₂-, -O-CH₂-O-, -O-CH₂-CH₂-O- and -CH₂-CH₂-NH-. An example of a substituted group is -CH₂-CH₂-N(CH₃)-.

In the context of the disclosure, the definition of A₁ or A₃ as a "five- to ten-membered monocyclic or fused polycyclic aromatic ring system" encompasses a C₆- or C₁₀-aromatic hydrocarbon group or a five- to ten-membered heterocyclic aromatic ring system. "Polycyclic" means preferably bicyclic.

In the context of the disclosure, the definition of R₂ as a "three- to six-membered monocyclic ring system" encompasses a C₆-aromatic hydrocarbon group, a five- to six-membered heterocyclic aromatic ring system and a three- to six-membered monocyclic aliphatic or heterocyclic ring system.

A C₆- or C₁₀-aromatic hydrocarbon group is typically phenyl or naphthyl, especially phenyl.

Preferably, but also depending on substituent definition, "five- to ten-membered heterocyclic aromatic ring systems" consist of 5 to 10 ring atoms of which 1-3 ring atoms are hetero atoms. Such heterocyclic aromatic ring systems may be present as a single ring system or as bicyclic or tricyclic ring systems; preferably as single ring systems or as benz-annelated ring systems. Bicyclic or tricyclic ring systems may be formed by annelation of two or more rings, or by a bridging atom, e.g. oxygen, sulfur, nitrogen. Examples of heterocyclic ring systems are: imidazo[2,1-b]thiazole, pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazolidine, isothiazole, isothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pteridine, and the corresponding benz-annelated heterocycles, e.g. indole, isoindole, coumarin, isoquinoline, quinoline and the like. Preferred heterocycles are: imidazo[2,1-b]thiazole, oxazole, isoxazole, thiazole, isothiazole, triazole, pyrrole, furane, tetrahydrofurane, pyridine, pyrimidine, imidazole or pyrazole.

In the context of the disclosure, three- to six-membered monocyclic aliphatic ring systems are typically cyclopropyl, cyclobutyl, cyclopentyl or cyclohexyl.

On account of asymmetrical carbon atom(s) that may be present in the compounds of formula (I) and compounds of formula (II), the compounds may exist in optically active form or in form of mixtures of optical isomers, e.g. in form of racemic mixtures or diastereomeric mixtures. All optical isomers and their mixtures, including racemic mixtures, are part of the present disclosure.

In one embodiment, the α7-nAChR agonist is a compound of formula (I) 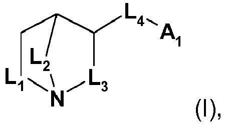 wherein
L₁ is -CH₂-; L₂ is -CH₂-CH₂-; and L₃ is -CH₂- or -CH(CH₃)-;
L₄ is a group selected from

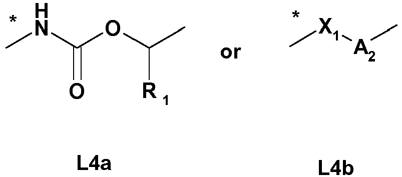

wherein the bond marked with the asterisk is attached to the azabicycloalkyl moiety; R₁ is hydrogen or C₁₋₄alkyl;
X₁ is -O- or -NH-;
A₂ is selected from

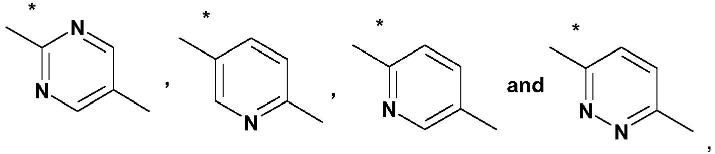

wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and
each R₂ independently is C₁₋₆alkyl, C₁₋₆halogenalkyl, C₁₋₆alkoxy, C₁₋₆halogenalkoxy or halogen.

In one embodiment, the α7-nAChR agonist is a compound of formula (I) 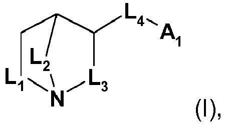 wherein
L₁ is -CH₂-; L₂ is -CH₂-CH₂-; and L₃ is -CH₂-;
L₄ is

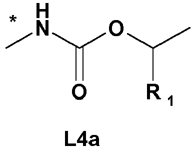

wherein the bond marked with the asterisk is attached to the azabicycloalkyl moiety; R₁ is hydrogen or C₁₋₄alkyl;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and
each R₂ independently is C₁₋₆alkyl, C₁₋₆halogenalkyl, C₁₋₆alkoxy, C₁₋₆halogenalkoxy or halogen.

In one embodiment, the α7-nAChR agonist is a compound of formula (I) 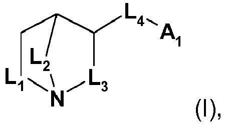 wherein
L₁ is -CH₂-; L₂ is -CH₂-CH₂-; and L₃ is -CH₂- or -CH(CH₃)-;
L₄ is

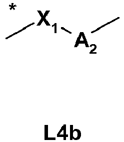

wherein the bond marked with the asterisk is attached to the azabicycloalkyl moiety; X₁ is -O- or -NH-;
A₂ is selected from

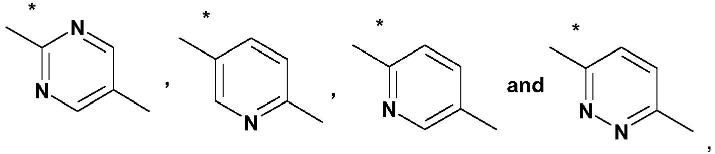

wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and
each R₂ independently is C₁₋₆alkyl, C₁₋₆halogenalkyl, C₁₋₆alkoxy, C₁₋₆halogenalkoxy or halogen.

In one embodiment, the α7-nAChR agonist is a compound of formula (I) 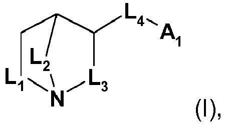 wherein
L₁ is -CH₂-CH₂-; L₂ is -CH₂-; and L₃ is -CH₂-CH₂-;
L₄ is

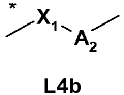

wherein the bond marked with the asterisk is attached to the azabicycloalkyl moiety; X₁ is -O- or -NH-;
A₂ is selected from

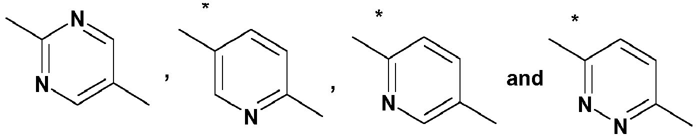

wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and
each R₂ independently is C₁₋₆alkyl, C₁₋₆halogenalkyl, C₁₋₆alkoxy, C₁₋₆halogenalkoxy or halogen.

Compounds of the group P1 are α7-nAChR agonists ; Group P1 is the group consisting of
A-1: (S)-(1-aza-bicyclo[2.2.2]oct-3-yl)-carbamic acid (S)-1-(2-fluoro-phenyl)-ethyl ester;
A-2: (R)-(1-aza-bicyclo[2.2.2]oct-3-yl)-carbamic acid (R)-1-(2-chloro-phenyl)-ethyl ester;
A-3: (S)-(1-aza-bicyclo[2.2.2]oct-3-yl)-carbamic acid (S)-1-phenyl-ethyl ester;
B-1: (R)-3-(5-phenyl-pyrimidin-2-yloxy)-1-aza-bicyclo[2.2.2]octane;
B-2: (R)-3-(5-p-tolyl-pyrimidin-2-yloxy)-1-aza-bicyclo[2.2.2]octane;
B-3: (R)-3-(5-(2-fluoro-4-methyl-phenyl)-pyrimidin-2-yloxy)-1-aza-bicyclo[2.2.2]octane;
B-4: (R)-3-(5-(3,4-dimethyl-phenyl)-pyrimidin-2-yloxy)-1-aza-bicyclo[2.2.2]octane;
B-5: (R)-3-(6-p-tolyl-pyridin-3-yloxy)-1-aza-bicyclo[2.2.2]octane;
B-6: (R)-3-(6-phenyl-pyridin-3-yloxy)-1-aza-bicyclo[2.2.2]octane;
B-7: (R)-3-(6-(3,4-dimethyl-phenyl)-pyridin-3-yloxy)-1-aza-bicyclo[2.2.2]octane;
B-8: (R)-3-[6-(2-fluoro-4-methyl-phenyl)-pyridazin-3-yloxy]-1-aza-bicyclo[2.2.2]octane;
B-9: (R)-3-[6-(4,5-dimethyl-2-fluoro-phenyl)-pyridazin-3-yloxy]-1-aza-bicyclo[2.2.2]octane;
B-10: (R)-3-[6-(3,4-dimethyl-phenyl)-pyridazin-3-yloxy]-1-aza-bicyclo[2.2.2]octane;
B-11: (R)-3-[6-(4-methyl-phenyl)-pyridazin-3-yloxy]-1-aza-bicyclo[2.2.2]octane;
B-12: (R)-3-[6-(2,5-difluoro-4-methyl-phenyl)-pyridazin-3-yloxy]-1-aza-bicyclo[2.2.2]octane;
B-13: (2S,3R)-3-[6-(1H-indol-5-yl)-pyridazin-3-yloxy]-2-methyl-1-aza-bicyclo[2.2.2]octane;
B-14: (2R,3S)-3-[6-(1H-indol-5-yl)-pyridazin-3-yloxy]-2-methyl-1-aza-bicyclo[2.2.2]octane;
B-15: (2S,3R)-3-[5-(1H-indol-5-yl)-pyrimidin-2-yloxy]-2-methyl-1-aza-bicyclo[2.2.2]octane;
B-16: (2R,3S)-3-[5-(1H-indol-5-yl)-pyrimidin-2-yloxy]-2-methyl-1-aza-bicyclo[2.2.2]octane; B-17: 3-[6-(1H-indol-5-yl)-pyridin-3-yloxy]-2-methyl-1-aza-bicyclo[2.2.2]octane;
B-18: (2S,3R)-2-methyl-3-[6-(5-methyl-thiophen-2-yl)-pyridazin-3-yloxy]-1-aza-bicyclo[2.2.2]octane;
B-19: 3-[6-(2,3-dimethyl-1H-indol-5-yl)-pyridazin-3-yloxy]-2-methyl-1-aza-bicyclo[2.2.2]octane;
B-20: trans-2-methyl-1-aza-bicyclo[2.2.2]oct-3-yl)-(6-phenyl-pyridin-3-yl)-amine;
B-21: trans -[6-(1H-indol-5-yl)-pyridin-3-yl]-(2-methyl-1-aza-bicyclo[2.2.2]oct-3-yl)-amine;
C-1: (4S,5R)-4-[5-(1H-indol-5-yl)-pyrimidin-2-yloxy]-1-aza-bicyclo[3.3.1]nonane;
C-2: 5-{2-[(4S,5R)-(1-aza-bicyclo[3.3.1]non-4-yl)oxy]-pyrimidin-5-yl}-1,3-dihydro-indol-2-one;
C-3: (4S,5R)-4-[6-(1H-indol-5-yl)-pyridin-3-yloxy]-1-aza-bicyclo[3.3.1]nonane;
C-4: (4S,5R)-4-[5-(1H-indol-5-yl)-pyridin-2-yloxy]-1-aza-bicyclo[3.3.1]nonane;
C-5: (4S,5R)-4-[6-(1H-indol-5-yl)-pyridazin-3-yloxy]-1-aza-bicyclo[3.3.1]nonane;
C-6: 5-{6-[(4S,5R)-(1-aza-bicyclo[3.3.1]non-4-yl)oxy]-pyridazin-3-yl}-1,3-dihydro-indol-2-one;
C-7: (1-aza-bicyclo[3.3.1]non-4-yl)-[5-(1H-indol-5-yl)-pyridin-2-yl]-amine;
C-8: (1-aza-bicyclo[3.3.1]non-4-yl)-[5-(1H-indol-5-yl)-pyrimidin-2-yl]-amine;
C-9: (1-aza-bicyclo[3.3.1]non-4-yl)-[6-(1H-indol-5-yl)-pyridin-3-yl]-amine;
C-10: (1-aza-bicyclo[3.3.1]non-4-yl)-[6-(1H-indol-5-yl)-pyridin-3-yl]-amine;
C-11: (1-aza-bicyclo[3.3.1]non-4-yl)-[5-(1H-indol-4-yl)-pyrimidin-2-yl]-amine;
C-12: (1-aza-bicyclo[3.3.1]non-4-yl)-[6-(1H-indol-5-yl)-pyridazin-3-yl]-amine;
D-1: 4-(5-phenyl-1,3,4-thiadiazol-2-yloxy)-1azatricyclo[3.3.1.1^{3,7}]decane having the formula

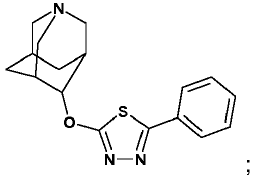

D-1a: (4S)-4-(5-phenyl-1,3,4-thiadiazol-2-yloxy)-1azatricyclo[3.3.1.1^{3,7}]decane;
D-1b: 4-(6-(1H-indol-5-yl)-pyridazin-3-yloxy)-1azatricyclo[3.3.1.1^{3,7}]decane;
D-1c: 4-(6-(1H-indol-5-yl)-pyridin-3-yloxy)-1azatricyclo[3.3.1.1^{3,7}]decane;
D-1d: 4-(5-(1H-indol-5-yl)-pyrimidin-2-yloxy)-1azatricyclo[3.3.1.1^{3,7}]decane;
D-2: 2-(6-phenylpyridazine-3-yl)octahydropyrrolo[3,4-c]pyrrole having the formula 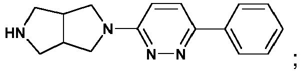
D-3: 5-[6-(5-methyl-hexahydro-pyrrolo[3,4-c]pyrrol-2-yl-pyridazin-3-yl1H-indole having the formula 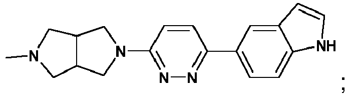
D-3a: 5-[6-(cis-5-methyl-hexahydro-pyrrolo[3,4-c]pyrrol-2-yl-pyridazin-3-yl1H-indole;
D-4: 5-[5-{6-methyl-3,6-diaza-bicyclo[3.2.0]hept-3-yl}-pyridin-2-yl]-1 H-indole having the formula 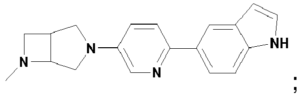
D-4a: 5-[5-{(1R,5R)-6-methyl-3,6-diaza-bicyclo[3.2.0]hept-3-yl}-pyridin-2-yl]-1 H-indole
D-5: 2-Methyl-5-(6-phenyl-pyridazin-3-yl)-octahydro-pyrrolo[3,4-c]pyrrole having the formula

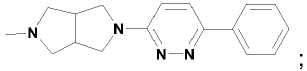

D-6: 5-{6-[1-azabicyclo[2.2.2]oct-3-yloxy]pyridazin-3-yl}-1H-indole;
D-6a: 5-{6-[(3R)-1-azabicyclo[2.2.2]oct-3-yloxy]pyridazin-3-yl}-1H-indole;
D-7: 5-{6-[1-azabicyclo[2.2.2]oct-3-yloxy]pyridazin-3-yl}-1,3-dihydro-indol-2-one;
D-7a: 5-{6-[(3R)1-azabicyclo[2.2.2]oct-3-yloxy]pyridazin-3-yl}-1,3-dihydro-indol-2-one;
D-8: N-(1-azabicyclo[2.2.2]oct-3-yl)-1H-indazole-3-carboxamide;
D-8a: N-((3R)-1-azabicyclo[2.2.2]oct-3-yl)-1H-indazole-3-carboxamide
D-8b: N-((3S)-1-azabicyclo[2.2.2]oct-3-yl)-1H-indazole-3-carboxamide
D-9: N-(1-azabicyclo[2.2.2]oct-3-yl)-5-(trifluoromethoxy)-1H-indazole-3-carboxamide;
D-9a: N-((3R)-1-azabicyclo[2.2.2]oct-3-yl)-5-(trifluoromethoxy)-1H-indazole-3-carboxamide;
D-9b: N-((3S)-1-azabicyclo[2.2.2]oct-3-yl)-5-(trifluoromethoxy)-1H-indazole-3-carboxamide;
D-10: N-(2-((3-pyridinyl)methyl)-1-azabicyclo[2.2.2]oct-3-yl)benzofuran-2-carboxamide;
D-10a: (2S,3R)-N-(2-((3-pyridinyl)methyl)-1-azabicyclo[2.2.2]oct-3-yl)benzofuran-2-carboxamide;
D-11: N-(2-((3-pyridinyl)methyl)-1-azabicyclo[2.2.2]oct-3-yl)-3,5-difluorobenzamide;
D-11a: (2S,3R)-N-(2-((3-pyridinyl)methyl)-1-azabicyclo[2.2.2]oct-3-yl)-3,5-difluorobenzamide;
D-11b: N-(2-((3-pyridinyl)methyl)-1-azabicyclo[2.2.2]oct-3-yl)-5-methylthiophene-2-carboxamide;
D-11c: (2S,3R)-N-(2-((3-pyridinyl)methyl)-1-azabicyclo[2.2.2]oct-3-yl)-5-methylthiophene-2-carboxamide;
D-11d: N-(2-((3-pyridinyl)methyl)-1-azabicyclo[2.2.2]oct-3-yl)-5-(2-pyridinyl)thiophene-2-carboxamide;
D-11e: (2S,3R)-N-(2-((3-pyridinyl)methyl)-1-azabicyclo[2.2.2]oct-3-yl)-5-(2-pyridinyl)thiophene-2-carboxamide;
D-12: 4-(5-methyloxazolo[4,5-b]pyridin-2-yl)-1,4-diazabicyclo[3.2.2]nonane;
D-13: [N-[(3R)-1-azabicyclo[2.2.2]oct-3-yl]-4-chlorobenzamide;
D-14: furo[2,3-c]pyridine-5-carboxylic acid (1-aza-bicyclo[2.2.2]oct-3-yl)-amide;
D-15: 2,3-dihydro-benzo[1,4]dioxine-6-carboxylic acid (1-aza-bicyclo[2.2.2]oct-3-yl)-amide;
D-16: 5-morpholin-4-yl-pentanoic acid (4-pyridin-3-yl-phenyl)-amide;
D-17: N-{4-[4-(2,4-dimethoxy-phenyl)-piperazin-1-yl]-butyl}-4-pyridin-2-yl-benzamide;
D-18: 1-[6-(4-fluorophenyl)pyridin-3-yl]-3-(4-piperidin-1-ylbutyl)-urea;
D-19: 7,8,9,10-tetrahydro-6,10-methano-6H-pyrazino-(2,3-h)(3)-benzazepine;
D-20: (2'R)-spiro-[1-azabicyclo[2.2.2]octane-3,2'(3'H)-furo[2,3-b]pyridine];
D-21: 1,4-Diaza-bicyclo[3.2.2]nonane-4-carboxylic acid 4-bromo-phenyl ester;
D-22: 3-[1-(2,4-Dimethoxy-phenyl)-meth-(E)-ylidene]-3,4,5,6-tetrahydro-[2,3']bipyridinyl;
D-23: 7-(2-Methoxy-phenyl)-benzofuran-2-carboxylic acid (1-aza-bicyclo[2.2.2]oct-3-yl)-amide;
D-24: N-methyl-1-{5-[3'H-spiro[4-azabicyclo[2.2.2]octane-2,2'-furo[2,3-b]pyridin]-5'-yl]-2-thienyl}methanamine having the formula

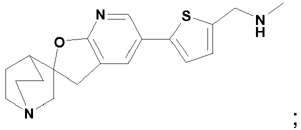

D-24a: N-methyl-1-{5-[(2R)-3'H-spiro[4-azabicyclo[2.2.2]octane-2,2'-furo[2,3-b]pyridin]-5'-yl]-2-thienyl}methanamine;
D-24b: N-methyl-1-{5-[(2S)-3'H-spiro[4-azabicyclo[2.2.2]octane-2,2'-furo[2,3-b]pyridin]-5'-yl]-2-thienyl}methanamine;
D-25a: 6-[(Anilinocarbonyl)amino]-N-[(3R)-1-azabicyclo[2.2.2]oct-3-yl]-1-benzothiophene-2-carboxamide;
D-25b: N-[(3R)-1-Azabicyclo[2.2.2]oct-3-yl]-6-({[(4-chlorophenyl)amino]carbonyl}amino)-1-benzothiophene-2-carboxamide;
D-25c: N-[(3R)-1-Azabicyclo[2.2.2]oct-3-yl]-6-({[(2-methoxyphenyl)amino]carbonyl}-amino)-1-benzothiophene-2-carboxamide;
D-25d: N-[(3R)-1-Azabicyclo[2.2.2]oct-3-yl]-6-({[(4-methoxyphenyl)amino]carbonyl}-amino)-1-benzothiophene-2-carboxamide;
D-25e: N-[(3R)-1-Azabicyclo[2.2.2]oct-3-yl]-6-({[(2-phenylethyl)amino]carbonyl}amino)-1-benzothiophene-2-carboxamide;
D-25f: N-[(3R)-1-Azabicyclo[2.2.2]oct-3-yl]-6-({[(3-cyanophenyl)amino]carbonyl}amino)-1-benziophene-2-carboxamide;
D-25g: N-[(3R)-1-Azabicyclo[2.2.2]oct-3-yl]-6-({[(3-bromophenyl)amino]carbonyl}amino)-1-benzothiophene-2-carboxamide;
D-25h: N-[(3R)-1-Azabicyclo[2.2.2]oct-3-yl]-6-({[(2-elhoxyphenyl)amino]carbonyl)amino)-1-benzothiophene-2-carboxamide;
D-25i: N-[(3R)-1-Azbicyclo[2.2.2]oct-3-yl]-6-({[(4-(dimethylamino)phenyl)amino]-carbonyl)amino)-1-benzothiophene-2-carboxamide;
D-25j: N-(3R)-1-Azabicyclo[2.2.2]oct-3-yl]-6-({[(2-nitrophenyl)amino]carbonyl}amino)-1-benzothiophene-2-carboxamide;
D-25k: N-[(3R)-1-Azabicyclo[2.2.2]oct-3-yl]-6-({[(2,6-difluorophenyl)amino]carbonyl}-amino)-1-benzothiophene-2-carboxamide;
D-25l: N-[(3R)-1-Azabicyclo[2.2.2]oct-3-yl]-6-({[(2,4-dichlorophenyl)amino]carbonyl}-amino)-1-benzothiophene-2-carboxamide;
D-25m: N-[(3R)-1-Azabicyclo[2.2.2]oct-3-yl]-6-[({[3-(trifluoromethyl)phenyl]amino]-carbonyl)amino]-1-benzothiophene-2-carboxamide;
D-25n: N-[(3R)-1-Azabicyclo[2.2.2]oct-3-yl]-6-({[(3,4,5-trimethoxyphenyl)amino]-carbonyl}amino)-1-benzothiophene-2-carboxamide;
D-25o: N-[(3R)-1-Azabicyclo[2.2.2]oct-3-yl]-6-[({[4-methoxy-3-(trifluoromethyl)phenyl]-amino}carbonyl)amino]-1-benzothiophene-2-carboxamide;
D-25p: N-{(3R)-1-Azabicyclo[2.2.2]oct-3-yl]-6-[({[3-methoxyphenyl]amino}carbonyl)-amino]-1-benzothiophene-2-carboxamide;
D-25q: N-[(3R)-1-Azabicyclo[2.2.2]oct-3-yl]-6-[({[3-trifluoromethoxyphenyl]amino}-carbonyl)-amino]-1-benzothiophene-2-carboxamide;
D-25r: N-[(3R)-1-Azabicyclo[2.2.2]oct-3-yl]-6-{[(tert-butylamino)carbonyl]amino}-1-benzothiophene-2-carboxamide;
D-25s: N-[(3R)-1-Azabicyclo[2.2.2]oct-3-yl]-6-{[(cyclohexylamino)carbonyl]amino}-1-benzothiophene-2-carboxamide;
D-25t: N-[(3R)-1-Azabicyclo[2.2.2]oct-3-yl]-6-[({[(1S)-1-phenylethyl]amino}carbonylamino]-1-benzothiophene-2-carboxamide;
D-25u: 7-[(Anilinocarbonyl)amino]-N-[(3R)-1-azabicyclo[2.2.2]oct-3-yl]-1-benzothiophene-2-carboxamide;
D-25v: N-[(3R)-1-Azabicyclo[2.2.2]oct-3-yl]-6-({[(4-methoxyphenyl)amino]carbonyl}-amino)-1-benzofuran-2-carboxamide;
D-26a: N-[4-(2-Thienyl)phenyl]-1-azabicyclo[2.2.2]octane-3-carboxamide;
D-26b: N-[4'-(Hydroxymethyl)-1,1'-biphenyl-4-yl]-1-azabicyclo[2.2.2]octane-3-carboxamide;
D-26c: N-(4'-Fluoro-1,1'-biphenyl-4-yl)-1-azabicyclo[2.2.2]octane-3-carboxamide;
D-26d: N-(4'-Methylsulfanyl-1,1'-biphenyl-4-yl)-1-azabicyclo[2.2.2]octane-3-carboxamide;
D-26e: 2-(1-Azabicyclo[2.2.2]oct-3-yl)-N-(4'-fluoro-1,1'-biphenyl-4-y1)acetamide;
D-26f: 2-(1-Azabicyclo[2.2.2]oct-3-yl)-N-(4'-methoxy-1,1'-biphenyl-4-yl)acetamide;
D-26g: 2-(1-Azabicyclo[2.2.2]oct-3-yl)-N-(4'-fluoro-1,1'-biphenyl-3-yl)acetamide;
D-26h: 2-(1-Azabicyclo[2.2.2]oct-3-yl)-N-(3'-nitro-1,1'-biphenyl-4-yl)acetamide;
D-26i: 2-(1-Azabicyclo[2.2.2]oct-3-yl)-N-[4'-(hydroxymethyl)-1,1'-biphenyl-3-yl]acetamide;
D-26j: 2-(1-Azabicyclo[2.2.2]oct-3-yl)-N-[4'-(bromomethyl)-1,1'-biphenyl-4-yl]acetamide;
D-26k: 2-(1-Azabicyclo[2.2.2]oct-3-yl)-N-[2'-(hydroxymethyl)-1,1'-biphenyl-3-yl]acetamide;
D-26l: N-[3'(Acetylamino)-1,1'-biphenyl-4-yl]-2-(1-azabicyclo[2.2.2]oct-3-yl)acetamide;
D-26m: (3R)-N-[2'-(Hydroxymethyl)-1,1'-biphenyl-4-yl]-1-azabicyclo[2.2.2]octane-3-carboxamide;
D-26n: (3R)-N-[4'-(Hydroxymethyl)-1,1'-biphenyl-4-yl]-1-azabicyclo[2.2.2]octane-3-carboxamide;
D-26o: (3S)-N-[4'(Hydroxymethyl)-1,1'-biphenyl-4-yl]-1-azabicyclo[2.2.2]octane-3-carboxamide;
D-26p: (3R)-N-[4'-(4-Morpholinyl)-1,1'-biphenyl-4-yl]-1-azabicyclo[2.2.2]octane-3-carboxamide;
D-26q: (3R)-N-[4'-(Hydroxymethyl)-3'-(methoxy)-1,1'-biphenyl-4-yl]-1-azabicyclo[2.2.2]-octane-3-carboxamide;
D-26r: Methyl 4'-{[(3S)-1-azabicyclo[2.2.2]oct-3-ylcarbonyl]amino}-1,1'-biphenyl-4-carboxylate;
D-26s: 4'-{[(3S)-1-Azabicyclo[2.2.2]oct-3-ylcarbonyl]amino}-1,1'-biphenyl-4-carboxylic Acid;
D-26t: (3R)-N-[4'-(Hydroxy-1-methylethyl)-1,1'-biphenyl-4-yl]-1-azabicyclo[2.2.2]-octane-3-carboxamide;
D-26u: (3R)-N-[4'-(Aminocarbonyl)-1,1'-biphenyl-4-yl]-1-azabicyclo[2.2.2]octane-3-carboxamide;
D-26v: (3R)-N-[4'-(Hydroxymethyl)-3-fluoro-1,1'-biphenyl-4-yl]-1-azabicyclo[2.2.2]octane-3-carboxamide;
D-26w: (4'-{[(3R)-1-Azabicyclo[2.2.2]oct-3-ylcarbonyl]amino}-1,1'-biphenyl-4-yl)methyl Methylcarbamate;
D-26x: (4'-{[(3R)-1-Azabicyclo[2.2.2]oct-3-ylcarbonyl]amino}-1,1'-biphenyl-4-yl)methyl Isopropylcarbamate;
D-26y: (4'-{[(3R)-1-Azabicyclo[2.2.2]oct-3-ylcarbonyl]amino}-1,1'-biphenyl-4-yl)methyl Ethylcarbamate;
D-26z: the free base form of a compound being selected from Examples No 26, 27, 28, 29, 30, 31, 32, 33, 34 and 35 of WO2003/078431;
D-27a: 2-(1-Azabicyclo[2.2.2]oct-3-yl)-N-(7-bromo-1-benzothien-2-yl)acetamide;
D-27b: 2-(1-Azabicyclo[2.2.2]oct-3-yl)-N-(6-bromo-1-benzothien-2-yl)acetamide;
D-27c: 2-(1-Azabicyclo[2.2.2]oct-3-yl)-N-(7-quinolinyl)acetamide;
D-27d: 2-(1-Azabicyclo[2.2.2]oct-3-yl)-N-(2-naphthyl)acetamide;
D-27e: 2-(1-Azabicyclo[2.2.2]oct-3-yl)-N-(8-nitro-2-naphthyl)acetamide;
D-28a: N-(1-Azabicyclo[2.2.2]oct-3-yl)-6-quinolinecarboxamide;
D-28b: N-(1-Azabicyclo[2.2.2]oct-3-yl)-2-phenazinecarboxamide;
D-28c: N-(1-Azabicyclo[2.2.2]oct-3-yl)-7-quinolinecarboxamide;
D-28d: N-[(3R)-1-Azabicyclo[2.2.2]oct-3-yl]-6-quinolinecarboxamide;
D-28e: N-(1-Azabicyclo[2.2.2]oct-3-yl)-2-ethyl-7-quinolinecarboxamide;
D-28f: N-(1-Azabicyclo[2.2.2]oct-3-yl)-2-ethyl-6-quinolinecarboxamide;
D-28g: N-(1-Azabicyclo[2.2.2]oct-3-yl)-2-methyl-7-quinolinecarboxamide;
D-28h: N-(1-Azabicyclo[2.2.2]oct-3-yl)-2-methyl-6-quinoliecarboxamide;
D-28i: N-(1-Azabicyclo[2.2.2]oct-3-yl)-4-methyl-6-quinolinecarboxamide;
D-28j: N-(1-Azabicyclo[2.2.2]oct-3-yl)-2-propyl-6-quinolinecarboxamide;
D-28k: N-(1-Azabicyclo[2.2.2]oct-3-yl)-2-ethyl-4-methyl-6-quinolinecarboxamide;
D-28l: N-(1-Azabicyclo[2.2.2]oct-3-yl)-2-propyl-7-quinolinecarboxamide;
D-28m: N-(1-Azabicyclo[2.2.2]oct-3-yl)-2-ethyl-4-methyl-7-quinolinecarboxamide;
D-28n: N-(1-Azabicyclo[2.2.2]oct-3-yl)-4-(tetrahydro-2H-pyran-2-yl)-6-quinoline-carboxamide;
D-28o: N-(1-Azabicyclo[2.2.2]oct-3-yl)-4-(tetrahydro-2H-pyran-2-yl)-7-quinoline-carboxamide;
D-28p: N-(1-Azabicyclo[2.2.2]oct-3-yl)-2-phenyl-6-quinolinecarboxamide;
D-28q: N-(1-Azabicyclo[2.2.2]oct-3-yl)-2-phenyl-7-quinolinecarboxamide;
D-29: (R)-7-chloro-N-(quinuclidin-3-yl)benzo[b]thiophene-2-carboxamide;
D-30a: 5-{5-[(endo)-8-azabicyclo[3.2.1]octan-3-yloxy]pyridin-2-yl}-1H-indole;
D-30b: 5-{5-[(exo)-8-azabicyclo[3.2.1]octan-3-yloxy]pyridin-2-yl}-1H-indole;
D-30c: 5-{5-[(endo)-8-methyl-8-aza-bicyclo[3.2.1]oct-3-yloxy]pyridin-2-yl}-1H-indole;
D-30d: 5-{5-[(exo)-8-methyl-8-aza-bicyclo[3.2.1]oct-3-yloxy]pyridin-2-yl}-1H-indole;D-30e: 4-{5-[(exo)-8-methyl-8-aza-bicyclo[3.2.1]oct-3-yloxy]pyridin-2-yl}-1H-indole; and
D-30f: 5-{6-[(exo)-8-methyl-8-aza-bicyclo[3.2.1]oct-3-yloxy]pyridin-3-yl}-1H-indole; wherein each of said compound is in free base form or in acid addition salt form.

In the present invention the alpha 7 nicotinic acetylcholine receptor agonist is (R)-3-(6-p-tolyl-pyridin-3-yloxy)-1-aza-bicyclo[2.2.2]octane in free base form or in acid addition salt form.

The compounds of formula (I) (e.g. compounds A-1 to A-3, B-1 to B-21 and C-1 to C-12) and their manufacture are known from WO2001/85727, WO2004/022556, WO2005/123732, WO2006/005608, WO2007/045478, WO2007/068476 and WO2007/068475, or can be prepared analogously to said references.
Compounds D-1 and D-1a can be prepared according to WO2008/058096.
Compounds D-2, D-3, D-3a, D-4, D-4a and D-5 (A-582941) can be prepared according to WO2005/028477. Compounds D-6, D-6a, D-7 and E7a can be prepared according to WO2006/065233 and/or WO2007/018738. Compounds D-8, D-8a, D-8b, D-9, D-9a and D-9b can be prepared according to WO2004/029050 and/or WO2010/043515.
Compounds D-10 and D-10a can be prepared according to WO2004/076449 and/or WO2009/018505. Compounds D-11, D-11a to D-11e can be prepared according to WO2004/076449 and/or WO2010/085724 and/or WO2010/056622. Compounds D-12 (CP-810123) and Compound D-19 (varenicline) are described in O'Donnell et al, J Med Chem, 2010, 53, 1222-1237. Compounds D-13 (PNU-282987), D-14 (PHA543613), D-21 (SSR-180771) and D-23 (ABBF) are described in Horenstein et al, Mol Pharmacol, 2008, 74, 1496-1511. Compounds D-15 (PHA568487), D-16 (WAY-317538), D-17 (WAY-264620), D-20 (AZD-0328) and D-22 (GTS-21) are described in Haydar et al, Current Topics in Medicinal Chemistry, 2010, 10, 144-152. Compound D-18 (WYE-103914) is described in Ghiron et al, J Med Chem, 2010, 53, 4379-4389. Compound D-24, D-24a and D-24b are described in WO2007/133155 and/or WO2009/066107. Compounds D-25a to D-25v are described in WO2004/013136. Compounds D-26a to D-26z are described in WO2003/078431. Compounds D-27a to D-27e are described in WO2003/078430. Compounds D-28a to D-28q are described in WO2003/043991. Compound D-29 is described in WO2003/055878. Compounds D-30a to D-30f are described in WO2007/137030.

### LMW α7-nAChR positive allosteric modulators:

### In one embodiment, the α7-nAChR activator used is an α7-nAChR positive allosteric modulator.

As used herein a "α7-nAChR positive allosteric modulator" is a compound that binds to a receptor comprising an α7-nAChR subunit in vivo and in vitro and is potentiating the activation of the receptor when its physiological ligand (i.e. acetylcholine) is binding. Potentiation can be measured by the method disclosed in WO2001/85727, i.e. a functional affinity assay at the homomeric α7-nAChR carried out with a rat pituitary cell line stably expressing the α7-nAChR. As read out, the calcium influx upon stimulation of the receptor compared to acetylcholine-binding alone is used. "α7-nAChR positive allosteric modulators" according to the disclosure typically induce calcium influx of at least 200% of the maximal influx evoked by acetylcholine with an EC₅₀ value of at least 5000nM; preferred α7-nAChR positive allosteric modulater induce calcium influx of at least 300% of the maximal influx evoked by acetylcholine with an EC₅₀ value of at least 1000nM; more preferred agonists induce calcium influx of at least 400% of the maximal influx evoked by epibatidine with an EC₅₀ value of at least 500nM.

In particular, preferred α7-nAChR positive allosteric modulators should be well absorbed from the gastrointestinal tract, should be sufficiently metabolically stable and possess favorable pharmacokinetic properties.

Further preferred α7-nAChR positive allosteric modulators bind in-vivo potently to α7-nAChRs whilst showing little affinity for other receptors, especially for other nAChRs, e.g. α 4β2 nAChR, for muscarinic acetylcholine receptors, e.g. M1, and/or the 5-HT₃ receptor. Further preferred α7-nAChR positive allosteric modulators cross the blood brain barrier effectively.

Preferred α7-nAChR positive allosteric modulators should be non-toxic and demonstrate few side-effects.

Furthermore, a preferred α7-nAChR positive allosteric modulator will be able to exist in a physical form that is stable, non-hygroscopic and easily formulated.

In one embodiment, the α7-nAChR positive allosteric modulator is a selective α7-nAChR positive allosteric modulator, i.e. is selective for a receptor comprising an α7-nAChR subunit, since such a positive allosteric modulator would be expected to cause fewer side effects than a non-selective positive allosteric modulator to a treated subject. A positive allosteric modulator being selective for a receptor comprising an α7-nAChR subunit has a functional affinity to such a receptor to a much higher degree, e.g. at least 10-fold affinity difference in EC₅₀ value, preferably at least 20-fold, more preferably at least 50-fold, compared to any other nicotinic acetylcholine receptor. To assess the affinity of the α7-nAChR positive allosteric modulator of the disclosure on other nicotinic acetylcholine receptors, the method disclosed in WO2001/85727 can be used, i.e. to assess the affinity on human neuronal α4β2 nAChR, a similar functional assay is carried out using a human embryonic kidney cell line stable expressing the human α4β2 subtype and to assess the activity of the compounds of the disclosure on the "ganglionic subtype" and the "muscle type" of nicotinic receptor, similar functional assays are carried out with a human embryonic kidney cell line stably expressing the human "ganglionic subtype" or a cell line endogenously expressing the human "muscle type" of nicotinic receptors.

In the last 12 years much effort has been focused on developing selective α7 nAChR positive allosteric modulators leading to the discovery of many different chemotypes displaying said selective activity. These efforts are summarized the review from Haydar et al (Current Topics in Medicinal Chemistry, 2010, 10, 144-152), which describes 11 compounds acting as α7 nAChR positive allosteric modulators belonging to seven different chemical families; i.e. XY-4083; PNU-120596, PHA-758454 and NS-1738; PHA-709829; SB-206553; LY-2087101, LY-1078733 and LY-2087133; compound 26; and A-867744 (compound designations taken from Haydar et al). In fact, at least one drug candidate having an α7 nAChR positive allosteric modulator mode of action obtained permission from the U.S. Food and Drug Administration to conduct clinical testing (i.e. XY-4083).

In one embodiment, the α7-nAChR positive allosteric modulator has a maximum molecular weight of 1500 daltons. In one embodiment, the α7-nAChR positive allosteric modulator has a maximum molecular weight of 1000 daltons. In one embodiment, the α 7-nAChR positive allosteric modulator has a maximum molecular weight of 800 daltons. In one embodiment, the α7-nAChR positive allosteric modulator has a maximum molecular weight of 500 daltons.

Compounds of the Group P5 are α 7-nAChR positive allosteric modulators; Group P5 is the group consisting of compounds
E-1: (Z)-N-(4-Chloro-phenyl)-3-(4-chloro-phenylamino)-2-(3-methyl-isoxazol-5-yl)-acrylamide (XY-4083);
E-2: 1-(5-Chloro-2,4-dimethoxy-phenyl)-3-(5-methyl-isoxazol-3-yl)-urea (PNU-120596);
E-3: 1-(5-Fluoro-2,4-dimethoxy-phenyl)-3-(5-trifluoromethyl-isoxazol-3-yl)-urea (PHA-758454);
E-4: 1-(5-Chloro-2-hydroxy-phenyl)-3-(2-chloro-5-trifluoromethyl-phenyl)-urea (NS-1738);
E-5: 4-(4-Chloro-phenyl)-2-(4-methoxy-phenyl)-5-methyl-2H-pyrazol-3-ylamine (PHA-709829);
E-6: 5-Methyl-3,5-dihydro-2H-pyrrolo[2,3-f]indole-1-carboxylic acid pyridin-3-ylamide (SB-206553);
E-7: [2-(4-Fluoro-phenylamino)-4-methyl-thiazol-5-yl]-thiophen-3-yl-methanone (LY-2087101);
E-8: [2-(4-Fluoro-phenylamino)-4-methyl-thiazol-5-yl]-p-tolyl-methanone (LY-1078733);
E-9: Benzo[1,3]dioxol-5-yl-[2-(4-fluoro-phenylamino)-4-methyl-thiazol-5-yl]-methanone (LY-2087133);
E-10: 4-Naphthalen-1-yl-3a,4,5,9b-tetrahydro-3H-cyclopenta[c]quinoline-8-sulfonic acid amide; and
E-11: 4-[5-(4-Chloro-phenyl)-2-methyl-3-propionyl-pyrrol-1-yl]-benzenesulfonamide (A-867744); wherein said compound is in free base form or in acid addition salt form.

In the present invention the alpha 7 nicotinic acetylcholine receptor agonist is (R)-3-(6-p-tolyl-pyridin-3-yloxy)-1-aza-bicyclo[2.2.2]octane in free base form or in acid addition salt form.

In yet another embodiment, the above disclosed compositions comprising an alpha 7 nicotinic acetylcholine receptor activator for the treatment of cognitive impairments, psychotic and/or neurodegenerative disorders in a group of patients selected according to the herein disclosed methods, comprises a second cognition enhancer or therapeutic compound, such as a conventional antipsychotic or an atypical antipsychotic.

In one embodiment the disclosure provides methods for predicting therapeutic responsiveness of a subject, *e.g.* a human subject, to alpha 7 nicotinic acetylcholine receptor activator treatment, based on the presence or absence of particular genetic markers in the subject to be treated. The term "predicting therapeutic responsiveness to alpha 7 nicotinic acetylcholine receptor activator treatment", as used herein, is intended to refer to an ability to assess the likelihood that treatment of a subject with an alpha 7 nicotinic acetylcholine receptor activator will or will not be clinically effective in (e.g., provide a measurable benefit to) the subject. In particular, such an ability to assess the likelihood that treatment will or will not be clinically effective is typically exercised before treatment with the alpha 7 nicotinic acetylcholine receptor activator is begun in the subject. However, it is also possible that such an ability to assess the likelihood that treatment will or will not be clinically effective can be exercised after treatment has begun but before an indicator of clinical effectiveness (*e.g.* an indicator of measurable benefit) has been observed in the subject.

The method comprises the steps of: I) obtaining the genotype of the individual at the genetic locus of the *CHRNA5* gene and/or the *CHRNA3* gene; II) identifying those individuals of step I) carrying the *CHRNA5* SNP rs55853698-T (SEQ ID NO. 1) or a SNP forming a haplotype with said SNPs and/or carrying the *CHRNA3* SNP rs6495308 (SEQ ID NO. 3/4) or the SNP rs1051730 (SEQ ID NO. 35/36) or a SNP forming a haplotype with said SNPs, wherein the homozygous presence of at least one of the said *CHRNA5* SNPs or SNP haplotypes is an indication that the individual will likely respond to the alpha 7 nicotinic acetylcholine receptor activator treatment, and wherein homozygous presence of the SNP rs6495308-T/T (SEQ ID NO. 4) is an indication that the individual will likely not respond to the alpha 7 nicotinic acetylcholine receptor activator treatment. Homozygous presence of the SNP rs6495308-C/C (SEQ ID NO. 3) or heterozygous presence of a SNP rs6495308-C/T genotype is an indication that the individual will likely respond to the alpha 7 nicotinic acetylcholine receptor activator treatment. The homozygous presence of the SNP rs1051730-C/C (SEQ ID NO. 35) is an indication that the individual will likely respond to the alpha 7 nicotinic acetylcholine receptor activator treatment.

Characterization of the *CHRNA5* and/or *CHRNA3* SNPs or obtaining genotype information of an individual at said loci may be accomplished by using any of the techniques well known in the art. For example, any of the regions of the genes may be sequenced. Any of the well-known methods for sequencing one or both strands of the *CHRNA5* and/or *CHRNA3* gene may be used in the methods of the disclosure, such as the methods described in, for example, U.S. Patent No. 5,075,216, Engelke et al. (1988) Proc. Natl. Acad. Sci. U.S.A. 85, 544-548 and Wong et al. (1987) Nature 330, 384-386; Maxim and Gilbert (1977) Proc. Natl. Acad. Sci. U.S.A. 74:560; or Sanger (1977)Proc. Natl. Acad. Sci. U.S.A. 74:5463. In addition, any of a variety of automated sequencing procedures can be utilized see, *e.g.,* Naeve, C.W et al. (1995) Biotechniques 19:448, including sequencing by mass spectrometry (see, *e.g.,* PCT International Publication No. WO 94/16101; Cohen et al. (1996) Adv. Chromatogr. 36:127-162; and Griffin et al. (1993) Appl. Biochem. Biotechnol. 38:147-159.

Determining the presence or absence of an *CHRNA5* and/or *CHRNA3* SNPs in a biological sample may be accomplished using any well known technique such as polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, deoxyribonucleic acid sequencing, restriction fragment length polymorphism analysis, haplotype analysis, serotyping, and combinations or sub-combinations thereof.

For example, a mRNA sample may be obtained from the subject and expression of mRNA(s) encoded by the *CHRNA5* and/or *CHRNA3* allele in the mRNA sample may be detected using standard molecular biology techniques, such as PCR analysis. A preferred method of PCR analysis is reverse transcriptase-polymerase chain reaction (RT-PCR). Other suitable systems for mRNA sample analysis include microarray analysis (*e.g.,* using Affymetrix's microarray system or Illumina's BeadArray Technology). In certain situations it may be possible to assay for the expression of an indicative marker allele of the *CHRNA5* or *CHRNA3* gene at the protein level, using a detection reagent that detects the protein product encoded by the mRNA of the biomarker. For example, if an antibody reagent is available that binds specifically to the *CHRNA5* or *CHRNA3* marker protein, and not to other proteins, then such an antibody can be used to detect the expression of the *CHRNA5* or *CHRNA3* marker protein in a cellular sample from the subject, or a preparation derived from the cellular sample, using standard antibody-based techniques known in the art, such as FACS analysis, ELISA and the like.

As indicated above, determining the presence or absence of an indicative marker allele of the *CHRNA5* or *CHRNA3* gene may include, for example, restriction fragment length polymorphism analysis. Restriction fragment length polymorphism analysis (RFLPS) is based on changes at a restriction enzyme site. Moreover, the use of sequence specific ribozymes (see, for example, U.S. Patent No. 5,498,531) may be used to score for the presence of a specific ribozyme cleavage site.

Another technique for determining the presence or absence of an indicative marker allele of the *CHRNA5* or *CHRNA3* gene or a SNP forming a haplotype with such an indicative allele involves hybridizing DNA segments which are being analyzed (target DNA) with a complimentary, labeled oligonucleotide probe as described in, for example, Wallace et al. (1981) Nucl. Acids Res. 9, 879-894. Since DNA duplexes containing even a single base pair mismatch exhibit high thermal instability, the differential melting temperature may be used to distinguish target DNAs that are perfectly complimentary to the probe from target DNAs that only differ by a single nucleotide. This method has been adapted to detect the presence or absence of a specific restriction site, as described in, for example, U.S. Patent No. 4,683,194. The method involves using an end-labeled oligonucleotide probe spanning a restriction site which is hybridized to a target DNA. The hybridized duplex of DNA is then incubated with the restriction enzyme appropriate for that site. Reformed restriction sites will be cleaved by digestion in the pair of duplexes between the probe and target by using the restriction endonuclease. The specific restriction site is present in the target DNA if shortened probe molecules are detected.

Other methods for determining the presence or absence of an indicative marker allele of the *CHRNA5* or *CHRNA3* gene or a SNP forming a haplotype with such an indicative allele include methods in which protection from cleavage agents is used to detect mismatched bases in RNA/RNA or RNA/DNA heteroduplexes (as described in, for example, Myers et al. (1985) Science 230:1242). In general, the art technique of "mismatch cleavage" starts by providing heteroduplexes formed by hybridizing (labeled) RNA or DNA containing the polymorphic sequence with potentially polymorphic RNA or DNA obtained from a sample. The double-stranded duplexes are treated with an agent which cleaves single-stranded regions of the duplex such as which will exist due to base-pair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels. See, for example, Cotton et al. (1988) Proc. Natl Acad Sci USA 85:4397; Saleeba et al. (1992) Methods Enzymol. 217:286-295. In a preferred embodiment, the control DNA or RNA can be labeled for detection.

In another embodiment, alterations in electrophoretic mobility may be used to determine the presence or absence of an indicative marker allele of the *CHRNA5* or *CHRNA3* gene or a SNP forming a haplotype with such an indicative allele. For example, single strand conformation polymorphism (SSCP) may be used to detect differences in electrophoretic mobility between various indicative marker allele of the *CHRNA5* or *CHRNA3* gene or a SNP forming a haplotype with such an indicative allele (as described in, for example, Orita et al. (1989) Proc Natl. Acad. Sci. USA: 86:276; Cotton (1993) Mutat Res 285:125-144; and Hayashi (1992) Genet Anal Tech Appl 9:73-79). Single-stranded DNA fragments of sample and control nucleic acids can be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet. 7:5).

In yet another embodiment, the movement of a nucleic acid molecule in polyacrylamide gels containing a gradient of denaturant is assayed using denaturing gradient gel electrophoresis (DGGE) (as described in, for example, Myers et al. (1985) Nature 313:495. When DGGE is used as the method of analysis, DNA can be modified to ensure that it does not completely denature, for example by adding a GC clamp of approximately 40 bp, of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for determining the presence or absence of an indicative marker allele of the *CHRNA5* or *CHRNA3* gene or a SNP forming a haplotype with such an indicative allele include, but are not limited to, selective oligonucleotide hybridization, selective amplification, or selective primer extension. For example, oligonucleotide primers may be prepared in which the polymorphic region is placed centrally and then hybridized to target DNA under conditions which permit hybridization only if a perfect match is found (Saiki et al. (1986) Nature 324:163; Saiki et al. (1989) Proc. Natl. Acad. Sci. USA 86:6230). Such allele specific oligonucleotides are hybridized to PCR amplified target DNA or a number of different polymorphisms when the oligonucleotides are attached to the hybridizing membrane and hybridized with labeled target DNA.

Another process for determining the presence or absence of an indicative marker allele of the *CHRNA5* or *CHRNA3* gene or a SNP forming a haplotype with such an indicative allele is the primer extension process which consists of hybridizing a labeled oligonucleotide primer to a template RNA or DNA and then using a DNA polymerase and deoxynucleoside triphosphates to extend the primer to the 5' end of the template. Resolution of the labeled primer extension product is then done by fractionating on the basis of size, e.g., by electrophoresis via a denaturing polyacrylamide gel. This process is often used to compare homologous DNA segments and to detect differences due to nucleotide insertion or deletion. Differences due to nucleotide substitution are not detected since size is the sole criterion used to characterize the primer extension product. Additional well known methods for SNP genotyping are:
Dynamic allele-specific hybridization (DASH) genotyping (Howell W., Jobs M., Gyllensten U., Brookes A. (1999) Dynamic allele-specific hybridization. A new method for scoring single nucleotide polymorphisms. Nat Biotechnol. 17(1):87-8).
SNP detection through molecular beacons (Abravaya K., Huff J., Marshall R., Merchant B., Mullen C., Schneider G., and Robinson J. (2003) Molecular beacons as diagnostic tools: technology and applications. Clin Chem Lab Med. 41:468-474).
High-density oligonucleotide SNP micrarrays (Rapley R., Harbron S. (Eds.) (2004) Molecular Analysis and Genome Discovery. Chichester. John Wiley & Sons Ltd.).
Flap endonucleases (The Invader assay for SNP genotyping. Mutat Res. 573(1-2):103-10).

In the event that a SNP is located in the promoter region, or another non-coding region having influence on the expression rate of the gene carrying said SNP, the mRNA or protein levels might be affected. In such a situation, the presence of a SNP can not be determined on the basis of the mRNA or protein sequence of said respective gene. However presence of such an indicative SNP could be determined indirectly by mRNA or Protein levels measurements. Hence, in another embodiment of the disclosure, the herein disclosed methods can comprise an additional or alternative step of determining the mRNA or protein level of a certain gene or gene product as an indirect determination method for the presence of an indicative SNP in the *CHRNA5* or *CHRNA3* gene. Falvella and co-workers have identified promoter polymorphisms and transcript levels of the *CHRNA5* gene (J. Natl. Cancer Inst 2010; 102:1366-1370, Vol. 102, Issue 17, September 8, 2010). In said publication it could be shown that the expression rate of the CHRNA5 gene, expressed as mRNA levels, of patients carrying the haplotype delTTC (formed by the SNPs rs3841324-Del (SEQ ID NO. 6), rs503464-T (SEQ ID NO. 10), rs55853698-T (SEQ ID NO. 1) and rs55781567-C (SEQ ID NO. 7) was 1.82 compared to 0.88 and 1.06 for patients carrying the InsTGG and InsATC haplotype, respectively. Consequently, the analysis of mRNA levels could be used as an indirect assay to determine the presence of the delTTC haplotype in said patients.

Haplotype analysis of one or more polymorphic sites around an indicative marker allele or SNP of the *CHRNA5* or *CHRNA3* gene may also be used for determining the presence or absence of additional indicative SNPs and may include, for example, use of family pedigrees, molecular techniques and/or statistical inference.

Moreover, any of the well-known methods for genotyping such SNPs (e.g., DNA sequencing, hybridisation techniques, PCR based assays, fluorescent dye and quenching agent-based PCR assay (Taqman PCR detection system), RFLP-based techniques, single strand conformational polymorphism (SSCP), denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, DHPLC-based techniques, oligonucleotide extension assays (OLA), extension based assays (ARMS, (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), pyrosequencing, protein truncation assay (PTT), immunoassays, haplotype analysis, and solid phase hydridization (dot blot, reverse dot blot, chips) are very well known in the art and described in, for example, Siitari, Nucleic acid diagnostics market, Technology Review 125/2002, ISDN 1239-758; Caplin (1999) Biochemica 1:5-8; Neville, (2002) BioTechniques 32:34-43; Underhill (197) Genome Res 7:996-1005; Oefner (2000) J Chromatogr B Biomed Sci Appl 739:345-55, and the patent publication No. U.S. 20010049586 and may be used in the methods of the disclosure.

Any suitable tissue sample obtained by biopsy or otherwise from the a subject afflicted with cognitive impairments, psychotic and/or neurodegenerative disorders may be used to determine the presence or absence of an indicative marker allele of the *CHRNA5* or *CHRNA3* gene or a SNP forming a haplotype with such an indicative allele. Techniques or methods for obtaining a biopsy from a subject are well known in the art. Isolating subcomponents of tissue samples (e.g., cells or RNA or DNA) may be accomplished using well known techniques in the art and those described in the Examples section below.

The presence, particularly the homozygous presence of the *CHRNA5* SNP rs55853698-T/T (SEQ ID NO. 1) or a SNP forming a haplotype with said SNPs is an indication that the individual will likely respond to the herein described alpha 7 nicotinic acetylcholine receptor activator treatment. The homozygous presence of the SNP rs6495308-T/T (SEQ ID NO. 4) is an indication that the individual will likely not respond to the alpha 7 nicotinic acetylcholine receptor activator treatment. Homozygous presence of the SNP rs6495308-C/C (SEQ ID NO. 3) or heterozygous presence of a SNP rs6495308-C/T genotype is an indication that the individual will likely respond to the alpha 7 nicotinic acetylcholine receptor activator treatment. The homozygous presence of the SNP rs1051730-C/C (SEQ ID NO. 35) is an indication that the individual will likely respond to the alpha 7 nicotinic acetylcholine receptor activator treatment.

Hence, the disclosure also relates to a therapeutic method of increasing the cognitive skills of an individual and/or treatment of individuals suffering from a cognitive impairment, psychotic and/or neurodegenerative disorder comprising the steps of: III) obtaining the genotype of the individual at the genetic locus of the *CHRNA5* gene as described above; IV) identifying those individuals of step III) carrying the *CHRNA5* SNP rs55853698-T (SEQ ID NO. 1), specifically those individuals being homozygous for the mentioned SNP variants, or a SNP forming a haplotype with said SNPs or a SNP in the same linkage disequilibrium with said SNPs and/or carrying the *CHRNA3* SNP rs6495308-C/C or T/C (SEQ ID NO. 3/4) or the SNP rs1051730-C/C (SEQ ID NO. 35) genotype or a SNP forming a haplotype with said SNPs and V) administering a therapeutic effective amount of an alpha 7 nicotinic acetylcholine receptor activator to those subject identified in step IV).

In an additional embodiment, the above described steps I) and III) further comprising the steps of: VI) obtaining a biological sample of said individual, wherein said sample is selected from the group consisting of blood, blood-derived product (such as buffy coat, serum, and plasma), lymph, urine, tear, saliva, cerebrospinal fluid, buccal swabs, sputum, hair roots, leukocyte sample or tissue samples or any combination thereof, and VII) contacting the biological sample of step VI) with a reagent or agent capable of detecting (as described in detail above) the CHRNA5 SNP rs55853698-T (SEQ ID NO.1), or the *CHRNA3* SNP rs6495308-C/C or T/C (SEQ ID NO. 3/4) or the SNP rs1051730 (SEQ ID NO. 35), or (v) a SNP forming a haplotype with said SNPs.

The reagent, agent or device with which the biological sample is contacted may be, for example, a PCR/sequencing primer(s), nucleotides and enzymes suitable for amplifying and/or sequencing and/or labeling the *CHRNA5* SNP rs55853698-T (SEQ ID NO. 1) or a SNP forming a haplotype with said SNPs and/or carrying the *CHRNA3* SNP rs6495308-C/C or T/C (SEQ ID NO. 3/4) or the SNP rs1051730 (SEQ ID NO. 35) or a SNP forming a haplotype with said SNPs present in the sample, an antibody capable of detecting one of the above mentioned SNPs or a SNP forming a haplotype with said SNPs in the sample, a restriction enzyme, and/or a microarray.

The term "therapeutically effective amount" in the context of administering an therapeutically effective amount as used herein typically refers to an amount of an active ingredient (e.g. alpha 7 nicotinic acetylcholine receptor activator and/or second cognition enhancer as described herein) which, when administered to a subject, is sufficient to provide a therapeutic benefit, e.g. is sufficient for treating cognitive impairments or dysfunctions, psychotic and/or neurodegenerative disorders, particularly for increasing the cognitive skills of an individual. In another aspect of the disclosure, the cognitive impairments or dysfunctions, psychotic and/or neurodegenerative disorders is a mental disease or a acquired deficit in one or more of memory function, problem solving, orientation and/or abstraction, particularly pronounced in verbal memory, executive functions, attention and vigilance, verbal fluency and motor speed. In an additional embodiment the cognitive impairments or dysfunctions, psychotic and/or neurodegenerative disorders is a mild cognitive impairment, Alzheimer's disease, Parkinson's disease dementia, dementia with Lewy Bodies, schizophrenia, vascular dementia, AIDS-dementia, senile dementia, mild cognitive impairment related to age (MCI), age associated memory impairment, autism, dementias in frontal lobe degenerations, stroke, basal ganglia degenerative disorders, multiple sclerosis, trauma, brain tumors, brain infections, hydrocephalus, depression, toxic or metabolic disorders and drug induced dementias.

Administration of an alpha 7 nicotinic acetylcholine receptor activator refers to the administration of an alpha 7 nicotinic acetylcholine receptor-agonist or -positive allosteric modulators, particularly to low molecular weight compounds being selected from group P1. Alternatively, the therapeutic method of increasing the cognitive skills of an individual and/or treatment of individuals suffering from a cognitive impairment, psychotic and/or neurodegenerative disorder comprises the step of administering the alpha 7 nicotinic acetylcholine receptor agonist as disclosed in formula (I), or a compound selected from the group P1.

"Pharmaceutically acceptable salts" are known in the field (e.g. S.M. Berge, et al, "Pharmaceutical Salts", J. Pharm. Sd., 1977, 66:1-19; and "Handbook of Pharmaceutical Salts, Properties, Selection, and Use", Stahl, RH., Wermuth, C.G., Eds.; Wiley-VCH and VHCA: Zurich, 2002). A pharmaceutically acceptable salt is intended to mean a salt of a free form that is not toxic, biologically intolerable, or otherwise biologically undesirable. Preferred pharmaceutically acceptable salts are those that are pharmacologically effective and suitable for contact with the tissues of patients without undue toxicity, irritation, or allergic response.

In yet another embodiment, in the disclosed methods of treatment a second cognition enhancer or a therapeutic compound useful for the treatment of cognitive impairments, psychotic and/or neurodegenerative disorders, such as a conventional antipsychotic or an atypical antipsychotic, can be administered. Preferably, a combination being a pharmaceutical composition or a combined pharmaceutical preparation is used. Such a pharmaceutical composition can be administered together, one after the other or separately in one combined unit dosage.

In another aspect of the disclosed methods, the alpha 7 nicotinic acetylcholine receptor activator dose to be administered is from about 1 mg to about 100 mg per day. Alternatively, the dose to be administered is from about 2 mg to about 100 mg, or about 3 mg to about 90 mg, or about 4 mg to about 80 mg, or about 5 mg to about 70 mg, or about 6 mg to about 60 mg, or about 7 mg to about 50 mg, or about 8 mg to about 40 mg, or about 9 mg to about 35 mg, or about 10 mg to about 30 mg per day, or about 5 mg to about 10 mg, or about 10 mg to about 15mg, or about 15 mg to about 20 mg, or about 20 mg to about 25 mg per day. In one embodiment of said aspects, the alpha 7 nicotinic acetylcholine receptor activator is an alpha 7 nicotinic acetylcholine receptor agonist.

Another embodiment of the disclosure relates to the use of an alpha 7 nicotinic acetylcholine receptor activator as described above for the treatment of a patient with cognitive impairments, psychotic and/or neurodegenerative disorders or condition in which alpha 7 nicotinic acetylcholine receptor activation plays a role or is implicated, wherein the patient being susceptible for the treatment with an alpha 7 nicotinic acetylcholine receptor activator has been selected according to the above described methods.

Furthermore, the disclosure relates to the use of at least one probe for detecting the *CHRNA5* SNP rs55853698-T (SEQ ID NO.1), or the *CHRNA3* SNP rs6495308-C/C or T/C (SEQ ID NO. 3/4), or the SNP rs1051730-C (SEQ ID NO. 35), or a SNP forming a haplotype with said SNPs or a SNP in the same linkage disequilibrium with said SNPs for determining whether an individual is responsive to (a) the treatment of cognitive impairments, psychotic and/or neurodegenerative disorders or condition in which alpha 7 nicotinic acetylcholine receptor activation plays a role or is implicated, or (b) the increase of cognitive skills by an alpha 7 nicotinic acetylcholine receptor activator. The skilled person is aware of methods and techniques how to design usable probes.

In another aspect, the disclosure relates to a kit comprising at least one probe for detecting the *CHRNA5* SNP rs55853698-T (SEQ ID NO.1), or the *CHRNA3* SNP rs6495308-C/C or T/C (SEQ ID NO. 3/4), or the SNP rs1051730 (SEQ ID NO. 35), or a SNP forming a haplotype with said SNPs. Preferably, said kit is a kit for diagnosing a responsiveness of an individual to the treatment of cognitive impairments, psychotic and/or neurodegenerative disorders or condition in which alpha 7 nicotinic acetylcholine receptor activation plays a role or is implicated by an alpha 7 nicotinic acetylcholine receptor activator, comprising VIII) means for detecting the (i) *CHRNA5* SNP rs55853698-T (SEQ ID NO.1), and/or (ii) the SNP rs16969968-G (SEQ ID NO. 37), and/or (iii) the *CHRNA3* SNP rs6495308-C/C or T/C (SEQ ID NO. 3/4), and/or (iv) the SNP rs1051730-C (SEQ ID NO. 35), and/or (v) a SNP forming a haplotype with said SNPs or a SNP in the same linkage disequilibrium with said SNPs, and IX) instructions how to use said kit.

An additional subject matter of the disclosure relates to the use of a kit, preferably the above disclosed kit, suitable for any of the above described methods or uses, wherein said kit comprises at least one probe for detecting the *CHRNA5* SNP rs55853698-T (SEQ ID NO.1), or the *CHRNA3* SNP rs6495308-C/C or T/C (SEQ ID NO. 3/4), or the SNP rs1051730-C (SEQ ID NO. 35), or a SNP forming a haplotype with said SNPs. In a related embodiment, the kit used as described above comprises oligonucleotide probes.

In another embodiment of the above described methods or uses the *CHRNA5*/*CHRNA3* SNP haplotype consists of at least two SNPs selected from the group comprising rs3841324 (SEQ IDs NO. 5 or 6), rs503464 (SEQ IDs NO. 9 or 10), rs55853698-T (SEQ ID NO. 1), rs55781567-C (SEQ ID NO. 7), rs56182392 (SEQ IDs NO. 11 or 12), rs77293642 (SEQ IDs NO. 13 or 14), rs67624739 (SEQ IDs NO. 15 or 16), rs142774214 (SEQ IDs NO. 17 or 18), rs60182379 (SEQ IDs NO. 19 or 20), rs77541452 (SEQ IDs NO. 21 or 22), rs72648882 (SEQ IDs NO. 23 or 24), rs144334096 (SEQ IDs NO. 25 or 26), rs114037126 (SEQ IDs NO. 27 or 28), rs140280786 (SEQ IDs NO. 29 or 30), rs147565924 (SEQ IDs NO. 31 or 32), rs16969968-G (SEQ ID NO. 37), rs6495308 (SEQ IDs NO. 3 or 4), rs1051730 (SEQ IDs NO. 35 or 36) and rs115662711 (SEQ IDs NO. 33 or 34). In another embodiment of the above described methods or uses the *CHRNA5* SNP haplotype consists of the indicative SNP rs55853698-T (SEQ ID NO. 1) and at least one additional SNP selected from the group consisting of rs3841324 (SEQ IDs NO.5 or 6), rs503464 (SEQ IDs NO. 9 or 10), rs55781567-C (SEQ ID NO. 7), rs56182392 (SEQ IDs NO. 11 or 12), rs77293642 (SEQ IDs NO. 13 or 14), rs67624739 (SEQ IDs NO. 15 or 16), rs142774214 (SEQ IDs NO. 17 or 18), rs60182379 (SEQ IDs NO. 19 or 20), rs77541452 (SEQ IDs NO. 21 or 22), rs72648882 (SEQ IDs NO. 23 or 24), rs144334096 (SEQ IDs NO. 25 or 26), rs114037126 (SEQ IDs NO. 27 or 28), rs140280786 (SEQ IDs NO. 29 or 30), rs147565924 (SEQ IDs NO. 31 or 32), rs16969968-G (SEQ ID NO. 37), rs6495308 (SEQ IDs NO. 3 or 4), rs1051730 (SEQ IDs NO. 35 or 36) and rs115662711 (SEQ IDs NO. 33 or 34). In a related embodiment, the SNP haplotype is selected from the haplotype delTTC and insATC formed by the SNPs rs3841324 (SEQ IDs NO. 5 and 6), rs503464 (SEQ IDs NO. 9 or 10), rs55853698-T (SEQ ID NO. 1) and rs55781567-C (SEQ ID NO. 7).

Actual dosage levels of the active agents in the pharmaceutical compositions of the present disclosure may be varied so as to obtain an amount of the active agent which is effective to achieve the desired therapeutic response for a particular patient, composition, and mode of administration, without being toxic to the patient. The selected dosage level will depend upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Administration of a "therapeutically effective dosage" of an alpha 7 nicotinic acetylcholine receptor activator comprised in the compositions of the disclosure can result in a decrease in severity of disease symptoms, an increase in frequency and duration of disease symptom-free periods, or a prevention of impairment or disability due to the disease affliction i.e. an improvement of cognitive skills.

A composition of the present disclosure can be administered by one or more routes of administration using one or more of a variety of methods known in the art. As will be appreciated by the skilled artisan, the route and/or mode of administration will vary depending upon the desired results. Routes of administration may include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrastemal injection and infusion. Alternatively, a composition can be administered by a nonparenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g. Sustained and Controlled Release Drug Delivery Systems, J.R. Robinson, ed., Marcel Dekker, Inc., New York, 1978.

Preferred therapeutic compositions are compositions for oral or transdermal administration.

A composition for enteral or parenteral administration is, for example, a unit dosage form, such as a sugar-coated tablet, a tablet, a capsule, a suppository or an ampoule.

The unit content of active ingredients in an individual dose need not in itself constitute a therapeutically effective amount, since such an amount can be reached by the administration of a plurality of dosage units. A composition according to the disclosure may contain, e.g., from about 10% to about 100%, preferably from about 20% to about 60%, of the active ingredients.

If not indicated otherwise, a pharmaceutical composition according to the disclosure is prepared in a manner known per se, e.g. by means of conventional mixing, granulating, sugar-coating, dissolving or lyophilizing processes. In preparing a composition for an oral dosage form, any of the usual pharmaceutical media may be employed, for example water, glycols, oils, alcohols, carriers, such as starches, sugars, or microcrystalline cellulose, diluents, granulating agents, lubricants, binders, disintegrating agents and the like. Because of their ease of administration, tablets and capsules represent the most advantageous oral dosage unit forms, in which case solid pharmaceutical carriers are obviously employed.

**SEQUENCES**

| SEQ ID NO. | SNP name | Sequence |
|---|---|---|
| 1 | CHRNA5 SNP rs55853698-T | CGGGAGCTGTGGCGCGGAGCGGCCCCTCTGCTGCGTCTGCCCTCGT TTTGTC |
| 2 | CHRNA5 SNP rs55853698-G | CGGGAGCTGTGGCGCGGAGCGGCCCCGCTGCTGCGTCTGCCCTCG TTTTGTC |
| 3 | CHRNA3 SNP rs6495308-C | GTAACTGTCTGATGGCAGGTTTGCTGCTGGGAGAGTAGAGAAGAGGT |
| | | TTGGG |
| 4 | CHRNA3 SNP rs6495308-T | GTAACTGTCTGATGGCAGGTTTGCTGTTGGGAGAGTAGAGAAGAGGT TTGGG |
| 5 | CHRNA5 SNP rs3841324-Ins | GAAGCTCCCGGCGCCTAGCCCCGCCCCTATTTCCCTCTGGCCCCGC CCAATCCTCGCCTTGTTCCTTTTGGCC |
| 6 | CHRNA5 SNP rs3841324-Del | GAAGCTCCCGGCGCCTAGCCCCGCCCAATCCTCGCCTTGTTCCTTTT GGCC |
| 7 | CHRNA5 SNP rs55781567-C | TTGTCTCACGACTCACACTCAGTGCTCCATTCCCCAAGAGTTCGCGTT CCCC |
| 8 | CHRNA5 SNP rs55781567-G | TTGTCTCACGACTCACACTCAGTGCTGCATTCCCCAAGAGTTCGCGTT CCCC |
| 9 | CHRNA5 SNP rs503464-A | ACAGCTCCCGCCGGGACAGCAGCCTCAGCCTAGCAGCTTCTGGCGG GCTCGG |
| 10 | CHRNA5 SNP rs503464-T | ACAGCTCCCGCCGGGACAGCAGCCTCTGCCTAGCAGCTTCTGGCGG GCTCGG |
| 11 | CHRNA5 SNP rs56182392-G | GGAGCTTCCACATGCGTCCCGAGCCCGCCAGAAGCTGCTAGGCTGA GGCTGC |
| 12 | CHRNA5 SNP rs56182392-A | GGAGCTTCCACATGCGTCCCGAGCCCACCAGAAGCTGCTAGGCTGA GGCTGC |
| 13 | CHRNA5 SNP rs77293642-C | CTGCTAGGCTGAGGCTGCTGTCCCGGCGGGAGCTGTGGCGCGGAG CGGCCCC |
| 14 | CHRNA5 SNP rs77293642-T | CTGCTAGGCTGAGGCTGCTGTCCCGGTGGGAGCTGTGGCGCGGAGC GGCCCC |
| 15 | CHRNA5 SNP rs67624739-Ins | GGAACAAGGCGAGGATTGGGCGGGGCCAGAGGGAAATAGGGGCGG GGCTAGGCGCCGGGAGCTTCCACATGCG |
| 16 | CHRNA5 SNP rs67624739-Del | GGAACAAGGCGAGGATTGGGCGGGGCTAGGCGCCGGGAGCTTCCA CATGCG |
| 17 | CHRNA5 SNP rs142774214-Ins | GGGCCAAAAGGAACAAGGCGAGGATTGGGCGGGGCCAGAGGG AAATAGGGGCGGGGCTAGGCGCCGGGAGCTT |
| 18 | CHRNA5 SNP rs142774214-Del | GGGCCAAAAGGAACAAGGCGAGGATTGGGCGGGGCTAGGCGCCGG GAGCTT |
| 19 | CHRNA5 SNP rs60182379-G | GGGGATCGGAGGCGGGGCTATGAATAGACCGGACTGGGCCAAAAGG AACAAG |
| 20 | CHRNA5 SNP rs60182379-T | GGGGATCGGAGGCGGGGCTATGAATATACCGGACTGGGCCAAAAGG AACAAG |
| 21 | CHRNA5 SNP rs77541452-A | GACAGGGTTGGACCAGAGGCGGGGCGAGGTGGGGAAAGGGTTGGT GGGGATC |
| 22 | CHRNA5 SNP rs77541452-G | GACAGGGTTGGACCAGAGGCGGGGCGGGGTGGGGAAAGGGTTGGT GGGGATC |
| 23 | CHRNA5 SNP rs72648882-G | TGTTATCTTAAGGTGTGCAGTGACTAGGCCAAGGACATCCGGTGGTT AGAGA |
| 24 | CHRNA5 SNP rs72648882-T | TGTTATCTTAAGGTGTGCAGTGACTATGCCAAGGACATCCGGTGGTTA GAGA |
| 25 | CHRNA5 SNP rs144334096-A | AGAGCAGACATCAATGAATAGCTGTTATCTTAAGGTGTGCAGTGACTA GGCC |
| 26 | CHRNA5 SNP rs144334096-T | AGAGCAGACATCAATGAATAGCTGTTTTCTTAAGGTGTGCAGTGACTA GGCC |
| 27 | CHRNA5 SNP rs114037126-C | AGCACAGAGCAGACATCAATGAATAGCTGTTATCTTAAGGTGTGCAGT GACT |
| 28 | CHRNA5 SNP rs114037126-T | AGCACAGAGCAGACATCAATGAATAGTTGTTATCTTAAGGTGTGCAGT GACT |
| 29 | CHRNA5 SNP rs140280786-A | CGGCTCCCTCTGCCACTGAAGTTTAAAGATAATTCGCCTGAATCCCTT CAGC |
| 30 | CHRNA5 SNP rs140280786-G | CGGCTCCCTCTGCCACTGAAGTTTAAGGATAATTCGCCTGAATCCCTT CAGC |
| 31 | CHRNA5 SNP rs147565924-A | TTCTTTGCAACCAGGTTTAGTTTTTCACAGATTTACTAGTCTGGAGGG TGGA |
| 32 | CHRNA5 SNP rs147565924-C | TTCTTTGCAACCAGGTTTAGTTTTTCCCAGATTTACTAGTCTGGAGGG TGGA |
| 33 | CHRNA5 SNP rs115662711-C | TGGCTCCATTGGATGTAATTTACATTCTTCCAGCTGTATTTGGACCTAA GAA |
| 34 | CHRNA5 SNP rs115662711-T | TGGCTCCATTGGATGTAATTTACATTTTTCCAGCTGTATTTGGACCTAA GAA |
| 35 | CHRNA3 SNP rs1051730-C | TGGGCCATCATCAAAGCCCCAGGCTACAAACACGACATCAAGTACAA CTGCT |
| 36 | CHRNA3 SNP rs1051730-T | TGGGCCATCATCAAAGCCCCAGGCTATAAACACGACATCAAGTACAA CTGCT |
| 37 | CHRNA5 SNP rs16969968-G | CTAGAAACACATTGGAAGCTGCGCTCGATTCTATTCGCTACATTACAA GACA |
| 38 | CHRNA5 SNP rs16969968-A | CTAGAAACACATTGGAAGCTGCGCTCAATTCTATTCGCTACATTACAA GACA |

### EXAMPLES

### General Methodology

To measure the effect of alpha 7 nicotinic acetylcholine receptor activator treatment on the cognitive skills of schizophrenic patients, an adequate cognitive test battery (CogState™) with short duration (as described in detail below) was applied allowing multiple time points measurements during the treatment period (Pietrzak, et al 2009; Maruff, et al 2009).

### Continuous Paired Associates Learning (CPAL) test

The CPAL task is a validated test that assesses visual episodic memory (associate learning). This test has been used previously in schizophrenia. Before beginning this task, the test supervisor reads the full instructions to the patient from the test supervisor script. On the CPAL test, participants must learn a series of associations between a set of difficult to verbalize pattern and location. In the presentation phase of the task, the pattern appears at the location and the subject is required to acknowledge that they have seen the pattern by touching the location at which it appears. At this stage of the task the patient will also see that there are two locations at which no target appears (distractor locations). Patterns are presented, in random order. However, once presented the pattern remains at the same location throughout the task. In the learning phase of the task, patients must place each of the eight patterns in their correct locations. They must do this in six rounds. For the first round, one of the patterns is presented in the centre location and the subject is required to remember the location at which it had been shown. They indicate the location by touching it. If they touch the incorrect location, a visual and audible signal occurs (a red cross appears over the location and a buzzer sound is presented). The patient is then required to choose a second location. This process continues until the patient has correctly placed all four of the targets in their correct locations. Once all eight patterns have been placed correctly, the second round begins. In the second round the patterns remain in the same locations, but their order of presentation in the centre of the screen is different to that of the first round (randomized). The process of placing each target in the correct location proceeds as it did in the first round. When the second round is complete, the same process is repeated as the rounds progress the number of errors made in placing the patterns in their correct locations reduces. Administration time is approximately 5 minutes in healthy volunteers. Patients with Schizophrenia would be expected to complete the task within about 12 minutes.

### NAB-Mazes test

The Neuropsychological Assessment Battery (NAB®) Mazes Test measures planning and foresight-aspects of executive function that are frequently impaired in patients with frontal lobe dysfunction. The measure quickly assesses planning and organizational skills through maze-tracing tasks that traditionally have been found to be sensitive to frontal lobe lesions. The NAB Mazes Test consists of seven mazes that become progressively more difficult, allowing the test supervisor to score the mazes based on completeness and completion time (NAB Neuropsychological Assessment Battery Mazes Test Kit; 2009, Robert A Stern Travis White).

### Matrics Consensus Cognitive Battery (MCCB, Matrics Assessment Inc.)

The Measurement and Treatment Research to Improve Cognition in schizophrenia (MATRICS) initiative determined that specific areas or domains of cognitive function should be investigated to determine the presence of treatment effects in schizophrenia. Seven domains of cognitive function were identified on the basis that they were reliably impaired in patients with chronic schizophrenia. These domains included speed of processing, attention/vigilance, working memory, verbal learning, visual learning, reasoning and problem solving, and social cognition.

### CPT-IP (Continuous Performance Test-Identical Pairs)

The pre-task on-screen instructions ask: "Is the card red?" The test supervisor will read full instructions to the subject from the test supervisor script. To begin the task, the test supervisor or subject must press the "Enter" key. A playing card is presented in the center of the screen. The card will flip over so it is face up. As soon as it does this the subject must decide whether the card is red or not. If it is red they should press "Yes", if it is not red they should press "No". The subject will practice until they reach the required number of responses, or until the practice period expires. Then, on screen instructions for the real test are presented. The test supervisor or subject must press the "Enter" key to begin the real test. The subject should be encouraged to work as quickly as they can and be as accurate as they can. For example, the subject should try not to press either the "Yes" or "No" key before a card flips over. If they make a mistake they will hear an error sound.

### Statistical methods for pharmacodynamic analyses

The assessment of activity was obtained from a statistical analysis of the post dose Area Under the Effect Curve (AUEC) 4-10 on CPAL as follows: variables were separately analyzed by means of a linear mixed effect model adjusted for the period-specific baseline value for the scale, the treatment group, the period, and the sequence as fixed effects, and for the patient as a random effect. The period-specific baseline value for the scale was obtained from the average of the period-specific Day -1 values and from the pre-dose value. The mean treatment difference (and its 95% CI) between each B-5 dose group and placebo was obtained from the model. The effect size was obtained by dividing the mean treatment difference (and its 95% CI) by the square root of twice the estimated variance of the residual error. Activity, as defined in the paragraph above, was assessed from the effect size of CPAL .

### Example 1: Study set up to identify if a subset of patients exist that respond to B-5 treatment.

In an attempt to identify such a subset of patients who respond to B-5 treatment, a study was conducted to investigate the relationship between the genetic variants rs55853698 (SEQ IDs NO. 1 and 2) in the *CHRNA5* gene and B-5 efficacy in the study. In the study, B-5 mono-fumarate was used in the form of hard gelatin capsules as described in Example E.

**Clinical Samples:** Genomic DNA from 29 individuals was extracted from whole blood according to the instructions from *Gentra Systems,* Inc. (Minneapolis, MN).

***Genotyping assay:*** A total of 29 DNA samples were genotyped for rs55853698 (SEQ IDs NO. 1 and 2) in the *CHRNA5* gene. Genotyping was performed using TaqMan Assays-by-Design and Assays-on-Demand (Applied Biosystems, Foster City, CA) on an ABI 7900 sequencer. Genotyping used 1 ng of genomic DNA according to manufacture's instruction.

***Statistical analysis:*** A mixed effect model including sequence, period and treatment as fixed effects, baseline value as covariate and subject as random effect was used. The effect size was determined by the difference in estimated means B-5-Placebo Treatment divided by the square root of twice the estimated variance of the residual error. Baseline disease severity was analyzed by ANCOVA (analysis of covariance) adjusted for age, gender, years of education, and smoking history.

***Results:*** The patients who are homozygous for the "T" variant of the *CHRNA5* SNP rs55853698-T/T (SEQ IDs No. 1) showed significant response to B-5 on "visual learning and memory" (CPAL) compared to placebo (2 mg: *p*=0.015, 15 mg: *p*=0.003, and 100 mg: *p*=0.024). The effect size in the three treatment arms was 0.63, 0.80 and 0.58, respectively. In contrast, the patients who are not homozygous for the "T" variant (G/T) or are homozygous for the "G" variant (G/G) of the *CHRNA5* SNP rs55853698 did not show any significant improvement on cognitive function (Table 1). The results show that the "TT" genotype of the *CHRNA5* SNP rs55853698-T (SEQ ID NO. 1) is a predictive marker of clinical response to B-5 in patients with schizophrenia.

**Table 1: Primary clinical endpoint CPAL assessment of CogState™ testbattery by CHRNA5 genotypes**

| Dose (mg) B-5 | rs55853698 genotype TT (n=18) | | rs55853698 genotype GG+GT (n=8) | |
|---|---|---|---|---|
| | effect size | P-value | effect size | P-value |
| 2 | 0.626 | 0.015 | - | - |
| 15 | 0.795 | 0.003 | -0.231 | 0.561 |
| 100 | 0.578 | 0.024 | -0.158 | 0.681 |

The key secondary clinical endpoint Matrics Consensus Cognitive Battery (MCCB) was evaluated regarding the B-5 efficacy in subpopulations determined by the *CHRNA5* variant (rs55853698). As summarized in Table 2, the patients who are homozygous for the "T" variant of the *CHRNA5* SNP rs55853698-T (SEQ ID NO. 1) showed greater improvement on "Reasoning and problem solving" (NAB-Mazes) compared to placebo. The effect size was 0.40, 0.50 and 0.32 in the 2 mg, 15 mg and 100 mg cohorts, respectively. In addition, the patients who are homozygous for the "T" variant of the *CHRNA5* SNP rs55853698-T (SEQ ID NO. 1) also showed greater improvement on "Attention and vigilance" (CPT-IP). The effect size was 0.47, 0.40 and 0.39 in the 2 mg, 15 mg and 100 mg cohorts, respectively. In contrast, the patients who are not homozygous for the "T" variant (G/T) or are homozygous for the "G" variant (G/G/) of the *CHRNA5* SNP rs55853698 did not show any significant improvement on cognitive function. The results show that the "TT" genotype of the *CHRNA5* SNP rs55853698-T (SEQ ID NO. 1) is a predictive marker of clinical response to B-5 in patients with schizophrenia.

**Table 2: Secondary clinical endpoint MCCB (Matrix consensus cognitive battery) by CHRNA5 genotypes**

| MATRICS: National Institute of Mental Health's initiative for Measurement and Treatment Research to Improve Cognition in schizophrenia initiative. | | | | |
|---|---|---|---|---|
| Dose (mg) B-5 | rs55853698 genotype TT (n=18) | | rs55853698 genotype GG+GT (n=8) | |
| | NAB mazes | | | |
| | effect size | P-value | effect size | P-value |
| 2 | 0.4 | 0.102 | -0.157 | 0.706 |
| 15 | 0.5 | 0.045 | -0.109 | 0.814 |
| 100 | 0.316 | 0.199 | -0.153 | 0.708 |

| | CPT-IP | | | |
|---|---|---|---|---|
| | effect size | P-value | effect size | P-value |
| 2 | 0.465 | 0.059 | - | - |
| 15 | 0.404 | 0.105 | 0.019 | 0.971 |
| 100 | 0.389 | 0.116 | -0.387 | 0.494 |

**Table 3: Primary clinical endpoint CPAL assessment of CogState™ testbattery by CHRNA3 (rs1051730) genotypes**

| Dose (mg) B-5 | rs1051730 genotype CC (n=19) | | rs1051730 genotype CT+TT (n=7) | |
|---|---|---|---|---|
| | effect size | P-value | effect size | P-value |
| 2 | 0.443 | 0.068 | 0.565 | 0.183 |
| 15 | 0.627 | 0.012 | -0.202 | 0.622 |
| 100 | 0.464 | 0.057 | -0.209 | 0.606 |

**Table 4: Primary clinical endpoint CPAL assessment of CogState™ testbattery by CHRNA3 (rs6495308) genotypes**

| Dose (mg) B-5 | rs6495308 genotype CC + CT (n=15) | | rs6495308 genotype TT (n=11) | |
|---|---|---|---|---|
| | effect size | P-value | effect size | P-value |
| 2 | 0.553 | 0.048 | 0.001 | 0.998 |
| 15 | 0.593 | 0.034 | -0.112 | 0.749 |
| 100 | 0.342 | 0.208 | 0.154 | 0.639 |

The following section discloses further aspects relating to this technology:

### Example A: Preparation of 5-chloro-2-(4-methylphenyl)pyridine:

Under nitrogen 2,5-dichloro-pyridine (40g, 270 mmol), 4-methylphenylboronic acid (39 g, 289 mmol) and bistriphenylphosphin-palladium(II) dichloride (1.14g; 1.6mmol) were suspended in water (258g) / THF (117g) for approx. 30 min at 35-55°C. A solution of tripotassium phosphate (143.4g , 676 mmol) in water (143g) was added at 35-55°C during approx. 60-120 min and 55°C was maintained for another approx. 30- 45 min.

More tripotassium phosphate (22.9g, 108mmol) in water (22.9g) was added over a period of approx. 30 min and the temperature was raised to 55-60°C to complete the reaction within another approx. 2h.

For extractive palladium removal a solution of cysteine (ca.16g) in water (115 g) was added to the reaction mixture at 60-55°C. After approx. 1h at 55°C the biphasic reaction mixture was clarified by filtration over a pad of cellflock filter aid (2-5g) and a THF/water mixture (110 g/75g) was used for rinsing. The layers of the combined filtrates were separated at 25°C and the salt containing water layer was extracted with THF (1x57g). The combined THF layers were diluted with ethanol 94% (195g) and concentrated by distillation under reduced pressure (300-200mbar) at a jacket temperature of 45°C in order to remove the bulk of THF (175-250g). To the remaining product solution further ethanol (97g) was added and at 45-55°C water (565g) was gradually added over a period of approx. 60min to induce and maintain crystallization. After 30 min the temperature was lowered to approx. 20°C in approx. 90 -120 min and after another hour at that temperature the solids were collected by filtration, washed with ethanol/water 1:2 and dried under reduced pressure to yield 5-chloro-2-(4-methylphenyl)pyridine (52.5g; 95% of theory; purity>95%; Pd <25ppm).

### Example B: Preparation of (R)-3-(6-(4-methylphenyl)-pyridin-3-yloxy)-1-aza-bicyclo[2.2.2]octane in free form and fumarate salt form

### Example B1: Formation of free form:

Under nitrogen, to 3R-quinuclidinol (43.8g, 0.34mol) in DMSO (792g) an approx. 20% THF solution of potassium tert-butoxide (210g, 0.375mol) was added and at approx. 40-45°C under reduced pressure the THF solvent was distilled off. The temperature of the reaction mixture was raised to 90°C and the solid 5-chloro-2-(4-methylphenyl)pyridine (61.2g, 0.30mol) was gradually added in at least 4 portions. The temperature was raised further to approx. 100-105°C and after at least another 3 hours at this temperature the reaction to (R)-3-(6-(4-methylphenyl)-pyridin-3-yloxy)-1-aza-bicyclo[2.2.2]octane was complete.

Water (150 g) was added to the reaction mixture at 60-25°C and the temperature was gradually lowered to approx. 20°C in approx. 60 min and additional water (210g) was added. After at least another 2 further hours at this temperature the fine solids were collected by filtration, washed successively with DMSO /water (approx. 322g; 2:1 mixture), water (500g) and water/ethanol (approx. 500g; 9:1 mixture) and dried at 60°C under reduced pressure to yield (R)-3-(6-(4-methylphenyl)-pyridin-3-yloxy)-1-aza-bicyclo[2.2.2]octane (56.3 g, 63% of theory).

### Example B2: Formation of fumarate salt form:

To a clear solution of (R)-3-(6-(4-methylphenyl)-pyridin-3-yloxy)-1-aza-bicyclo [2.2.2] octane (39.6g; 0.135 mol) and fumaric acid (16.4g, 0.141 mol) in ethanol (330g) / water (21g) at 65°C tert.-butylmethylether (142.5g) was added and the reaction mixture was cooled to 23°C in approx. 60min. Further tert.-butylmethylether (170.6g) was added. After at least another 2 hours the solids were collected by filtration, washed with ethanol/tert.butylmethylether (153 g; 1.1 mixture) and dried at 55-60°C under reduced pressure to yield (R)-3-(6-(4-methylphenyl)-pyridin-3-yloxy)-1-aza-bicyclo[2.2.2]octane hydrogenfumarate (43.8g, 79% of theory).

### Example C: Preparation of (R)-3-(6-(4-methylphenyl)-pyridin-3-yloxy)-1-aza-bicyclo[2.2.2]octane in free form and fumarate salt form Example C1: Formation of free form:

Under nitrogen to 3R-quinuclidinol (41.4g, 0.325mol) in DMSO (320g) a solution of 5-chloro-2-(4-methylphenyl)pyridine (51g, 0.250mol) in toluene (201g) was added. The temperature was raised gradually to approx. 100-105°C while residual water, if any, was removed by refluxing under reduced pressure at a water trap for ca. 45 min. Over a period of approx. 90 min an approx. 20% THF solution of potassium tert-butoxide (158.8g, 0.283mol) was continuously added while gradually the THF solvent distills off.

After another 2-5 hours at approx. 100-105°C the reaction to (R)-3-(6-(4-methylphenyl)-pyridin-3-yloxy)-1-aza-bicyclo[2.2.2]octane was complete.

Water (293g) was added to the reaction mixture at 60-25°C. The layers were separated and the toluene layer was washed with water (2x42g). The toluene solution was dried at ca. 60°C by refluxing under reduced pressure at a water trap for ca. 45-60 min.

### Example C2: Formation of fumarate salt form:

To the toluene solution of Example C1, at ca. 50-55°C, a slurry of fumaric acid (26.1g, 0.9eq) in EtOH 94% (22g) and toluene (97g) was gradually added. Further toluene (97g) was added for rinsing and after another ca. 30-60 min at 55°C the temperature was gradually lowered to approx. 20°C in approx. 120-180 min. After at least another 1 hour the solids were collected by filtration, washed with water saturated toluene (2x104g) and dried at 60°C under reduced pressure to yield (R)-3-(6-(4-methylphenyl)-pyridin-3-yloxy)-1-aza-bicyclo[2.2.2]octane hydrogenfumarate (84.8g; 82% of theory, based on amount of 5-chloro-2-(4-methylphenyl)pyridine used in Example C1).

### Example D: Preparation of mono-fumarate salt of (R)-3-(6-(4-methylphenyl)-pyridin-3-yloxy)-1-aza-bicyclo[2.2.2]octane in crystalline form

500 mg of (R)-3-(6-(4-methylphenyl)-pyridin-3-yloxy)-1-aza-bicyclo[2.2.2]octane in free base form were suspended in 20 ml isopropyl alcohol. A stochiometric amount of fumaric acid was added. The resulting solution was stirred at ambient temperature for 14 hours. The precipitate was collected by filtration.

### Example D1: Preparation of mono-fumarate salt of (R)-3-(6-(4-methylphenyl)-pyridin-3-yloxy)-1-aza-bicyclo[2.2.2]octane in crystalline form by seeded crystallization

7.3 g mono-fumarate of (R)-3-(6-(4-methylphenyl)-pyridin-3-yloxy)-1-aza-bicyclo [2.2.2]octane (purity >98%; prepared as described e.g. in Example C2) was dissolved in ethanol (42.9g)/isopropanol (8.5g)/water (7.2g) at about 50°C, clarified by filtration and added at this temperature gradually over a period of about 8 hours to filtered tertiary-butylmethylether (118.4g) at a temperature of about 50°C. After about 25% of the filtrate was added, an ultrasonificated suspension of seed crystals of the mono-fumarate of (R)-3-(6-(4-methylphenyl)-pyridin-3-yloxy)-1-aza-bicyclo[2.2.2]octane (6mg, prepared e.g. as described in Example C2) in isopropanol (0.1 ml) was added to induce crystallization. The product suspension was maintained for another 1 hour at 50°C and cooled to 0°C within 8 hours. After another 1 hour at this temperature the solids were isolated by filtration, washed with isopropanol/tertiary-butylmethylether (40ml, 1:1 mixture) and dried at about 50°C under reduced pressure to yield the mono-fumarate of (R)-3-(6-(4-methylphenyl)-pyridin-3-yloxy)-1-aza-bicyclo[2.2.2]octane (5.85 g; 81% of theory; purity >99.5%).

### Example E: Hard Capsules

Hard gelatin capsules, each comprising as active ingredient 0.5, 5 or 25 mg of the mono-fumarate of (R)-3-(6-(4-methylphenyl)-pyridin-3-yloxy)-1-aza-bicyclo[2.2.2]octane can be prepared as follows:

| Ingredient for capsule fill | % (w/w) for 0.5 mg capsules | % (w/w) for 5 mg capsules | % (w/w) for 25 mg capsules |
|---|---|---|---|
| Mono-fumarate of (R)-3-(6-(4-methylphenyl)-pyridin-3-yloxy)-1-aza-bicyclo[2.2.2]octane | 0.46 | 4.65 | 23.23 |
| Lactose monohydrate | 65.24 | 61.05 | 42.47 |
| Microcrystalline cellulose | 25.00 | 25.00 | 25.00 |
| Hypromellose | 2.50 | 2.50 | 2.50 |
| Sodium croscarmellose | 6.00 | 6.00 | 6.00 |
| Colloidal silicon dioxide | 0.30 | 0.30 | 0.30 |
| Magnesium stearate | 0.50 | 0.50 | 0.50 |
| Purified water* | q.s. | q.s. | q.s. |

| | | | |
|---|---|---|---|
| * removed during processing | | | |

Preparation process: Mono-fumarate of (R)-3-(6-(4-methylphenyl)-pyridin-3-yloxy)-1-aza-bicyclo[2.2.2]octane, lactose monohydrate, microcrystalline cellulose, a portion of sodium croscarmellose and hypromellose were dry mixed in a high shear mixer bowl, and granulating fluid (purified water) added. Once the granulation was complete, the wet granules were dried in a fluid bed drier and the dry granules were milled. The remaining sodium croscarmellose and colloidal silicon dioxide were passed through a suitable sieve and added to the dried granular material and blended in a suitable blending shell. This was achieved by co-sieving the sodium croscarmellose and the colloidal silicon dioxide with a portion of the milled granules through a suitable sieve into the blending shell. Similarly, the required amount of sieved magnesium stearate was added to the bulk granule and then mixed in the same blending shell. This final blend was encapsulated into capsules using automated equipment. Weight ratio of capsule fill to empty capsule shells was 2:1.

### Example F: Tablets

### Example F1: Film-coated tablet

Film-coated tablets containing e.g. 0.5 mg of the mono-fumarate of (R)-3-(6-(4-methylphenyl)-pyridin-3-yloxy)-1-aza-bicyclo[2.2.2]octane may be prepared as follows:

### Preparation of pre-mix:

Weigh-in mono-fumarate of (R)-3-(6-(4-methylphenyl)-pyridin-3-yloxy)-1-aza-bicyclo[2.2.2]octane (e.g. approx. 0.7%) and maize starch (e.g. approx. 13%), mix in a tumble blender (approx 100-300 rotations), pass through a sieve of approx. 0.25-1.0 mm mesh-size. Mix in a tumble blender (approx. 100-300 rotations).

### Preparation of final blend:

To above pre-mix add microcrystalline cellulose (e.g. approx. 25%), sprayed lactose (e.g. approx. 68%), sodium-carboxymethylcellulose XL (e.g. approx. 2%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations).

Add the sodium-stearyl-fumarate (e.g. approx. 1.5%) through a handsieve at approx. 0.5-1.0 mm mesh-size and mix in a tumble blender (approx. 30-150 rotations).

### Compression:

On a rotary press compress the above final blend to cores of approx. 100mg, using the dosage specific tooling (e.g. approx. 6mm, round, curved).

### Coating:

Prepare a suspension in water with basic coating premixes black, red, yellow and/or white. Coat the above obtained cores in a perforated coating pan, and dry.

### Example F2: Bilayer film-coated tablet

Bilayer film-coated tablets containing e.g. 2.5 mg of the mono-fumarate of (R)-3-(6-(4-methylphenyl)-pyridin-3-yloxy)-1-aza-bicyclo[2.2.2]octane may be prepared as follows:

### Final active blend:

Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations).

Add the Na-stearyl-fumarate (e.g. approx. 3%) through a handsieve at approx. 0.5-10mm and mix in a tumble blender (approx 30-150 rotations).

### Final placebo blend:

Weigh-in microcrystalline cellulose (e.g. approx. 26%), sprayed lactose (e.g. approx. 69%), sodium-carboxymethylcellulose XL (e.g. approx. 1.9%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations).

Add the sodium-stearyl-fumarate (e.g. approx. 3%) through a handsieve at approx. 0.5-1.0 mm and mix in a tumble blender (approx 30-150 rotations).

### Compression:

On a rotary press compress the above final blends to a bilayer tablet-core of approx. 100mg with one placebo layer (approx. 77.5mg) and one active layer (approx. 22.5mg), using the dosage specific tooling (e.g. approx. 6mm, round, curved).

### Coating:

Prepare a suspension in water with basic coating premixes black, red, yellow and/or white. Coat the above obtained cores in a perforated coating pan, and dry.

### Example F3: Film-coated tablet

Film-coated tablets containing e.g. 50 mg of the mono-fumarate of (R)-3-(6-(4-methylphenyl)-pyridin-3-yloxy)-1-aza-bicyclo[2.2.2]octane may be prepared as follows:

### Final blend:

Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations).

Add the sodium-stearyl-fumarate (e.g. approx. 3%) through a handsieve at approx. 0.5-10mm and mix in a tumble blender (approx. 30-150 rotations).

### Compression:

Compress the above final blend on a rotary press to cores, using the dosage specific tooling (e.g. approx. 15^{∗}5.9mm, round, curved).

### Coating:

Prepare a suspension in water with basic coating premixes black, red, yellow and/or white. Coat the above obtained cores in a perforated coating pan, and dry.

### References

1. Chini B, Raimond E, Elgoyhen AB, Moralli D, Balzaretti M, Heinemann S (1994). - Molecular cloning and chromosomal localization of the human alpha 7-nicotinic receptor subunit gene (CHRNA7). Genomics 19: 379-381.
2. Freedman R, Hall M, Adler LE, Leonard S (1995). - Evidence in postmortem brain tissue for decreased numbers of hippocampal nicotinic receptors in schizophrenia. Biological.Psychiatry 38: 22-33.
3. Freedman R, Coon H, Myles-Worsley M, Orr-Urtreger A, Olincy A, Davis A, Polymeropoulos M, Holik J, Hopkins J, Hoff M, Rosenthal J, Waldo MC, Reimherr F, Wender P, Yaw J, Young DA, Breese CR, Adams C, Patterson D, Adler LE, Kruglyak L, Leonard S, Byerley W (1997). - Linkage of a neurophysiological deficit in schizophrenia to a chromosome 15 locus. - Proceedingsof the National Academy of Sciences of the United States of America. 94: 587-592.
4. Goldberg TE, Goldman RS, Burdick KE, Malhotra AK, Lencz T, Patel RC, Woerner MG, Schooler NR, Kane JM, Robinson DG (2007). - Cognitive improvement after treatment with second-generation antipsychotic medications in first-episode schizophrenia: is it a practice effect? - Archives of General Psychiatry 64: 1115-1122.
5. Green MF (1996). - What are the functional consequences of neurocognitive deficits in schizophrenia?. American Journal of Psychiatry 153: 321-330.
6.Green MF (2007). - Cognition, drug treatment, and functional outcome in schizophrenia: a tale of two transitions. American Journal of Psychiatry 164: 992-994.
7. Harvey PD, Green MF, Keefe RS, Velligan DI (2004). - Cognitive functioning in schizophrenia: a consensus statement on its role in the definition and evaluation of effective treatments for the illness. Journal of Clinical Psychiatry 65: 361-372.
8. Keefe RS, Bilder RM, Davis SM, Harvey PD, Palmer BW, Gold JM, Meltzer HY, Green MF, Capuano G, Stroup TS, McEvoy JP, Swartz MS, Rosenheck RA, Perkins DO, Davis CE, Hsiao JK, Lieberman JA, CATIE I, - Neurocognitive Working Group (2007). - Neurocognitive effects of antipsychotic medications in patients with chronic schizophrenia in the CATIE Trial. - Archives of General Psychiatry 64: 633-647.
9. Leonard S, Gault J, Hopkins J, Logel J, Vianzon R, Short M, Drebing C, Berger R, Venn D, Sirota P, Zerbe G, Olincy A, Ross RG, Adler LE, Freedman R (2002). - Association of promoter variants in the alpha7 nicotinic acetylcholine receptor subunit gene with an inhibitory deficit found in schizophrenia. Archives of General Psychiatry 59: 1085-1096.

## Claims

1. Composition comprising an alpha 7 nicotinic acetylcholine receptor agonist for use in treatment of cognitive impairments, psychotic and/or neurodegenerative disorders in a selected patient population,
wherein the patient population is selected on the basis of having at least one indicative SNP of the human acetylcholine receptor subunit alpha-5 (*CHRNA5)* gene or the human acetylcholine receptor subunit alpha-3 (*CHRNA3)* gene,
wherein any of a homozygous genotype of rs55853698-T/T or a SNP forming a haplotype with said SNP, are indicative SNPs of the CHRNA5 gene,
wherein any of a homozygous genotype of rs1051730-C/C, a homozygous genotype of rs6495308-C/C, a heterozygous genotype of rs6495308-C/T, or a SNP forming a haplotype with any of said SNPs are indicative SNPs of the CHRNA3 gene, and
wherein the alpha 7 nicotinic acetylcholine receptor agonist is (R)-3-(6-p-tolyl-pyridin-3-yloxy)-1-aza-bicyclo[2.2.2]octane in free base form or in acid addition salt form.

2. Composition for use according to claim 1, wherein the cognitive impairments, psychotic and/or neurodegenerative disorders are conditions in which alpha 7 nicotinic acetylcholine receptor activation plays a role or is implicated, and
wherein the cognitive impairments, psychotic and/or neurodegenerative disorders are selected from the group consisting of mild cognitive impairment, Alzheimer's disease, Parkinson's disease dementia, dementia with Lewy Bodies, schizophrenia, vascular dementia, AIDS-dementia, senile dementia, mild cognitive impairment related to age (MCI), age associated memory impairment, autism, dementias in frontal lobe degenerations, stroke, basalganglia degenerative disorders, multiple sclerosis, trauma, brain tumors, brain infections, hydrocephalus, depression, toxic or metabolic disorders and drug induced dementias.

3. Composition for use according to claim 1, wherein the cognitive impairment, psychotic and/or neurodegenerative disorder is schizophrenia.

4. Composition for use according to any of the claims 1 to 3, wherein the selected patients have a homozygous genotype of rs55853698-T/T (SEQ. ID NO. 1).

5. Composition for use according to any one of claims 1 to 3, wherein the selected patients have a homozygous genotype of rs1051730-C/C (SEQ. ID NO. 35).

6. Composition for use according to any one of claims 1 to 3, wherein the selected patients have a homozygous genotype of rs6495308-C/C (SEQ. ID NO. 3).

7. Composition for use according to any one of claims 1 to 3, wherein the selected patients have a heterozygous genotype of rs6495308-C/T.

8. Composition for use according to any one of claims 1 to 7, comprising the alpha 7 nicotinic acetylcholine receptor agonist in association with a pharmaceutical carrier or diluent.

9. Composition according to any one of claims 1 to 8, further comprising a second cognition enhancer or a therapeutic compound useful for the treatment of cognitive impairments, psychotic and/or neurodegenerative disorders,
wherein the second cognition enhancer or therapeutic compound useful for the treatment of cognitive impairments, psychotic and/or neurodegenerative disorders is a conventional antipsychotic or an atypical antipsychotic.

10. Composition for use according to any one of claims 1 to 9, wherein the alpha 7 nicotinic acetylcholine receptor agonist dose to be administered is from about 2 mg to about 100 mg per day.

11. A method for predicting therapeutic responsiveness of an individual or a group of individuals to alpha 7 nicotinic acetylcholine receptor agonist treatment for increasing the cognitive skills and/or treatment of a cognitive impairment, psychotic and/or neurodegenerative disorder comprising the steps of:
I. obtaining the genotype of the individual at the genetic locus of the *CHRNA5* gene; and
II. identifying those individuals of step I. carrying the *CHRNA5* SNP rs55853698-T/T (SEQ ID NO. 1) or a SNP forming a haplotype with said SNP,
wherein the presence of at least one of the SNP or SNP haplotype listed in step II) above is an indication that the individual will likely respond to the alpha 7 nicotinic acetylcholine receptor agonist treatment, and
wherein the alpha 7 nicotinic acetylcholine receptor agonist is (R)-3-(6-p-tolyl-pyridin-3-yloxy)-1-aza-bicyclo[2.2.2]octane in free base form or in acid addition salt form.

12. A method for predicting therapeutic responsiveness of an individual or a group of individuals to alpha 7 nicotinic acetylcholine receptor agonist treatment for increasing the cognitive skills and/or treatment of a cognitive impairment, psychotic and/or neurodegenerative disorder comprising the steps of:
I. obtaining the genotype of the individual at the genetic locus of the *CHRNA3* gene; and
II. identifying those individuals of step I) carrying one or more of the *CHRNA3* SNPs rs6495308 (SEQ ID NO. 3/4) and the SNP rs1051730 (SEQ ID NO. 35/36), wherein a homozygous presence of the SNP rs6495308-C/C (SEQ ID NO. 3), heterozygous presence of a SNP rs6495308-C/T, or homozygous presence of the SNP rs1051730-C/C (SEQ ID NO. 35) genotype is an indication that the individual will likely respond to the alpha 7 nicotinic acetylcholine receptor agonist treatment,
wherein the alpha 7 nicotinic acetylcholine receptor agonist is (R)-3-(6-p-tolyl-pyridin-3-yloxy)-1-aza-bicyclo[2.2.2]octane in free base form or in acid addition salt form.

13. Use of at least one probe for detecting
(i) the CHRNA5 SNP rs55853698-T/T (SEQ ID NO.1), or
(ii) the CHRNA3 SNP rs6495308-C/T or-C/C (SEQ ID NO. 3/4), or
(iii) the CHRNA3 SNP rs1051730-C/C (SEQ ID NO. 35/36), or
(iv) a SNP forming a haplotype with said SNPs for determining whether an individual is responsive to
(a) the treatment of a cognitive impairment or a psychotic and/or neurodegenerative disorder with (R)-3-(6-p-tolyl-pyridin-3-yloxy)-1-aza-bicyclo[2.2.2]octane, or
(b) the increase of cognitive skills by (R)-3-(6-p-tolyl-pyridin-3-yloxy)-1-aza-bicyclo[2.2.2]octane.

14. The method or use of any of claims 11 to 13, wherein the cognitive impairment, psychotic and/or neurodegenerative disorder is schizophrenia.

## Patentansprüche

1. Zusammensetzung, umfassend einen Agonisten von nikotinischem Acetylcholinrezeptor-Alpha-7 zur Verwendung bei der Behandlung von kognitiven Beeinträchtigungen, psychotischen und/oder neurodegenerativen Störungen in einer ausgewählten Patientenpopulation,
wobei die Patientenpopulation auf der Basis des Aufweisens von mindestens einem indikativen SNP des Gens der humanen Acetylcholinrezeptor-Untereinheit Alpha-5 (*CHRNA5*) oder des Gens der humanen Acetylcholinrezeptor-Untereinheit Alpha-3 (*CHRNA3*) ausgewählt wird,
wobei jedwedes von einem homozygoten Genotyp von rs55853698-T/T oder einem SNP, der mit dem genannten SNP einen Haplotyp bildet, indikative SNPs des CHRNA5-Gens sind,
wobei jedwedes von einem homozygoten Genotyp von rs1051730-C/C, einem homozygoten Genotyp von rs6495308-C/C, einem heterozygoten Genotyp von rs6495308-C/T oder einem SNP, der einen Haplotyp mit beliebigen der genannten SNPs bildet, indikative SNPs des CHRNA3-Gens sind, und
wobei es sich bei dem Agonisten von nikotinischem Acetylcholinrezeptor-Alpha-7 um (R)-3-(6-p-Tolylpyridin-3-yloxy)-1-aza-bicyclo[2.2.2]octan in Form der freien Base oder in Form eines Säureadditionssalzes handelt.

2. Zusammensetzung zur Verwendung nach Anspruch 1, wobei die kognitiven Beeinträchtigungen, psychotischen und/oder neurodegenerativen Störungen Zustände sind, bei denen die Aktivierung von nikotinischem Acetylcholinrezeptor-Alpha-7 eine Rolle spielt oder impliziert wird, und
wobei die kognitiven Beeinträchtigungen, psychotischen und/oder neurodegenerativen Störungen ausgewählt sind aus der Gruppe bestehend aus leichter kognitiver Beeinträchtigung, Morbus Alzheimer, Demenz bei Morbus Parkinson, Demenz mit Lewy-Körperchen, Schizophrenie, vaskulärer Demenz, AIDS-Demenz, seniler Demenz, altersbedingter milder kognitiver Beeinträchtigung (MCI), altersbedingter Gedächtnisstörung, Autismus, Demenz bei Degenerationen des Frontallappens, Schlaganfall, degenerativer Basalganglien-Störungen, multipler Sklerose, Trauma, Hirntumoren, Hirninfektionen, Hydrozephalus, Depression, toxischen oder metabolischen Störungen und Arzneimittel-verursachter Demenz.

3. Zusammensetzung zur Verwendung nach Anspruch 1, wobei es sich bei der kognitiven Beeinträchtigung, der psychotischen und/oder der neurodegenerativen Störung um Schizophrenie handelt.

4. Zusammensetzung zur Verwendung nach beliebigen der Ansprüche 1 bis 3, wobei die ausgewählten Patienten einen homozygoten Genotyp von rs55853698-T/T (SEQ. ID NO. 1) aufweisen.

5. Zusammensetzung zur Verwendung nach einem der Ansprüche 1 bis 3, wobei die ausgewählten Patienten einen homozygoten Genotyp von rs1051730-C/C (SEQ. ID NO. 35) aufweisen.

6. Zusammensetzung zur Verwendung nach einem der Ansprüche 1 bis 3, wobei die ausgewählten Patienten einen homozygoten Genotyp von rs6495308-C/C (SEQ. ID NO. 3) aufweisen.

7. Zusammensetzung zur Verwendung nach einem der Ansprüche 1 bis 3, wobei die ausgewählten Patienten einen heterozygoten Genotyp von rs6495308-C/T aufweisen.

8. Zusammensetzung zur Verwendung nach einem der Ansprüche 1 bis 7, umfassend den Agonisten von nikotinischem Acetylcholinrezeptor-Alpha-7 in Assoziation mit einem pharmazeutischen Träger oder Verdünnungsmittel.

9. Zusammensetzung nach einem der Ansprüche 1 bis 8, ferner umfassend einen zweiten Kognitionsverstärker oder eine therapeutische Verbindung, die zur Behandlung von kognitiven Beeinträchtigungen, psychotischen und/oder neurodegenerativen Störungen nützlich ist,
wobei der zweite Kognitionsverstärker oder die therapeutische Verbindung, die zur Behandlung von kognitiven Beeinträchtigungen, psychotischen und/oder neurodegenerativen Störungen nützlich ist, ein herkömmliches Antipsychotikum oder ein atypisches Antipsychotikum ist.

10. Zusammensetzung zur Verwendung nach einem der Ansprüche 1 bis 9, wobei die zu verabreichende Dosis des Agonisten von nikotinischem Acetylcholinrezeptor-Alpha-7 etwa 2 mg bis etwa 100 mg pro Tag beträgt.

11. Verfahren zur Vorhersage des therapeutischen Ansprechens eines Individuums oder einer Gruppe von Individuen auf die Behandlung mit einem Agonisten von nikotinischem Acetylcholinrezeptor-Alpha-7 zur Steigerung der kognitiven Fähigkeiten und/oder zur Behandlung einer kognitiven Beeinträchtigung, einer psychotischen und/oder neurodegenerativen Störung, umfassend die folgenden Schritte:
I. Erhalten des Genotyps des Individuums am genetischen Lokus des *CHRNA5-*Gens; und
II. Identifizieren derjenigen Individuen von Schritt I., die den *CHRNA5*-SNP rs55853698-T/T (SEQ ID NO. 1) oder einen SNP tragen, der mit dem genannten SNP einen Haplotyp bildet,
wobei das Vorhandensein von mindestens einem von dem in Schritt II) oben aufgeführten SNP oder SNP-Haplotyp ein Hinweis darauf ist, dass das Individuum wahrscheinlich auf die Behandlung mit einem Agonisten von nikotinischem Acetylcholinrezeptor-Alpha-7 ansprechen wird, und
wobei es sich bei dem Agonisten von nikotinischem Acetylcholinrezeptor-Alpha-7 um (R)-3-(6-p-Tolylpyridin-3-yloxy)-1-aza-bicyclo[2.2.2]octan in Form der freien Base oder in Form eines Säureadditionssalzes handelt.

12. Verfahren zur Vorhersage des therapeutischen Ansprechens eines Individuums oder einer Gruppe von Individuen auf die Behandlung mit einem Agonisten von nikotinischem Acetylcholinrezeptor-Alpha-7 zur Steigerung der kognitiven Fähigkeiten und/oder Behandlung einer kognitiven Beeinträchtigung, einer psychotischen und/oder neurodegenerativen Störung, umfassend die folgenden Schritte:
I. Erhalten des Genotyps des Individuums am genetischen Lokus des *CHRNA3*-Gens; und
II. Identifizieren derjenigen Individuen von Schritt I), die eines oder mehrere der *CHRNA3*-SNPs rs6495308 (SEQ ID NO. 3/4) und des SNP rs1051730 (SEQ ID NO. 35/36) tragen, wobei eine homozygote Anwesenheit des SNP rs6495308-C/C (SEQ ID NO. 3), heterozygote Anwesenheit eines SNP rs6495308-C/T, oder homozygote Anwesenheit des SNP rs1051730-C/C (SEQ ID NO. 35)-Genotyps ein Hinweis darauf ist, dass das Individuum wahrscheinlich auf die Behandlung mit dem Agonisten von nikotinischem Acetylcholinrezeptor-Alpha-7 ansprechen wird,
wobei es sich bei dem Agonisten von nikotinischem Acetylcholinrezeptor-Alpha-7 um (R)-3-(6-p-Tolylpyridin-3-yloxy)-1-aza-bicyclo[2.2.2]octan in Form der freien Base oder in Form eines Säureadditionssalzes handelt.

13. Verwendung mindestens einer Sonde zum Nachweisen
(i) des CHRNA5-SNP rs55853698-T/T (SEQ ID NO.1) oder
(ii) des CHRNA3-SNP rs6495308-C/T oder -C/C (SEQ ID NO. 3/4) oder
(iii) des CHRNA3-SNP rs1051730-C/C (SEQ ID NO. 35/36) oder
(iv) eines SNP, der mit den genannten SNPs einen Haplotyp bildet, um zu bestimmen, ob ein Individuum anspricht auf
(a) die Behandlung einer kognitiven Beeinträchtigung oder einer psychotischen und/oder neurodegenerativen Störung mit (R)-3-(6-p-Tolylpyridin-3-yloxy)-1-aza-bicyclo[2.2.2]octan oder
(b) die Steigerung der kognitiven Fähigkeiten durch (R)-3-(6-p-Tolylpyridin-3-yloxy)-1-aza-bicyclo-[2.2.2]octan.

14. Verfahren oder Verwendung von beliebigen der Ansprüche 11 bis 13, wobei es sich bei der kognitiven Beeinträchtigung, der psychotischen und/oder der neurodegenerativen Störung um Schizophrenie handelt.

## Revendications

1. Composition comprenant un agoniste du récepteur nicotinique alpha-7 de l'acétylcholine pour une utilisation dans le traitement de troubles cognitifs, de troubles psychotiques et/ou neurodégénératifs dans une population sélectionnée de patients,
où la population de patients est sélectionnée sur la base de la possession d'au moins un SNP indicatif du gène de la sous-unité alpha-5 du récepteur de l'acétylcholine humain (*CHRNA5*) ou du gène de la sous-unité alpha-3 du récepteur de l'acétylcholine humain (*CHRNA3*),
où l'un quelconque parmi un génotype homozygote de rs55853698-T/T ou un SNP formant un haplotype avec ledit SNP, sont des SNP indicatifs du gène *CHRNA5,*
où l'un quelconque parmi un génotype homozygote de rs1051730-C/C, un génotype homozygote de rs6495308-C/C, un génotype hétérozygote de rs6495308-C/C, ou un SNP formant un haplotype avec l'un quelconque parmi lesdits SNP, sont des SNP indicatifs du gène *CHRNA3,* et
où l'agoniste du récepteur nicotinique alpha-7 de l'acétylcholine est le (R)-3-(6-p-tolylpyridin-3-yloxy)-1-azabicyclo[2.2.2]octane sous forme de base libre ou sous forme de sel d'addition d'acide.

2. Composition pour une utilisation selon la revendication 1, où les troubles cognitifs, les troubles psychotiques et/ou neurodégénératifs sont des conditions dans lesquelles l'activation du récepteur nicotinique alpha-7 de l'acétylcholine joue un rôle ou est impliquée, et
où les troubles cognitifs, les troubles psychotiques et/ou neurodégénératifs sont choisis dans le groupe constitué par un trouble cognitif léger, la maladie d'Alzheimer, la démence liée à la maladie de Parkinson, la démence à corps de Lewy, la schizophrénie, la démence vasculaire, la démence liée au SIDA, la démence sénile, le trouble cognitif léger lié à l'âge (MCI), le trouble de la mémoire lié à l'âge, l'autisme, les démences des dégénérescences du lobe frontal, les accidents vasculaires cérébraux, les troubles de dégénérescence des noyaux gris centraux, la sclérose en plaques, les traumatismes, les tumeurs cérébrales, les infections cérébrales, l'hydrocéphalie, la dépression, les troubles toxiques ou métaboliques et les démences induites par les drogues.

3. Composition pour une utilisation selon la revendication 1, où le trouble cognitif, le trouble psychotique et/ou neurodégénératif est la schizophrénie.

4. Composition pour une utilisation selon l'une quelconque des revendications 1 à 3, où les patients sélectionnés possèdent un génotype homozygote de rs55853698-T/T (SEQ ID n° 1).

5. Composition pour une utilisation selon l'une quelconque des revendications 1 à 3, où les patients sélectionnés possèdent un génotype homozygote de rs1051730-C/C (SEQ ID n° 35).

6. Composition pour une utilisation selon l'une quelconque des revendications 1 à 3, où les patients sélectionnés possèdent un génotype homozygote de rs6495308-C/C (SEQ ID n° 3).

7. Composition pour une utilisation selon l'une quelconque des revendications 1 à 3, où les patients sélectionnés possèdent un génotype homozygote de rs6495308-C/T.

8. Composition pour une utilisation selon l'une quelconque des revendications 1 à 7, comprenant l'agoniste du récepteur nicotinique alpha-7 de l'acétylcholine en association avec un véhicule ou diluant pharmaceutique.

9. Composition selon l'une quelconque des revendications 1 à 8, comprenant en outre un deuxième améliorateur de cognition ou un composé thérapeutique utile pour le traitement des troubles cognitifs, des troubles psychotiques et/ou neurodégénératifs,
où le deuxième améliorateur de cognition ou composé thérapeutique utile pour le traitement des troubles cognitifs, des troubles psychotiques et/ou neurodégénératifs est un antipsychotique classique ou un antipsychotique atypique.

10. Composition pour une utilisation selon l'une quelconque des revendications 1 à 9, où la dose d'agoniste du récepteur nicotinique alpha-7 de l'acétylcholine à administrer va d'environ 2 mg à environ 100 mg par jour.

11. Méthode de prédiction de la réactivité thérapeutique d'un individu ou d'un groupe d'individus vis-à-vis d'un traitement par un agoniste du récepteur nicotinique alpha-7 de l'acétylcholine afin d'augmenter les performances cognitives et/ou le traitement d'un trouble cognitif, d'un trouble psychotique et/ou neurodégénératif, comprenant les étapes consistant à :
I. obtenir le génotype de l'individu au niveau du locus génétique du gène *CHRNA5* ; et
II. identifier les individus de l'étape I) portant le SNP rs55853698-T/T (SEQ ID n° 1) de *CHRNA5* ou un SNP formant un haplotype avec ledit SNP,
où la présence d'au moins un parmi le SNP ou l'haplotype de SNP énuméré dans l'étape II) ci-dessus constitue une indication selon laquelle l'individu répondra probablement à un traitement par un agoniste du récepteur nicotinique alpha-7 de l'acétylcholine, et
où l'agoniste du récepteur nicotinique alpha-7 de l'acétylcholine est le (R)-3-(6-p-tolylpyridin-3-yloxy)-1-azabicyclo[2.2.2]octane sous forme de base libre ou sous forme de sel d'addition d'acide.

12. Méthode de prédiction de la réactivité thérapeutique d'un individu ou d'un groupe d'individus vis-à-vis d'un traitement par un agoniste du récepteur nicotinique alpha-7 de l'acétylcholine afin d'augmenter les performances cognitives et/ou le traitement d'un trouble cognitif, d'un trouble psychotique et/ou neurodégénératif, comprenant les étapes consistant à :
I. obtenir le génotype de l'individu au niveau du locus génétique du gène *CHRNA3* ; et
II. identifier les individus de l'étape I) portant un ou plusieurs parmi les SNP rs6495308 (SEQ ID n° 3/4) de *CHRNA3* et SNP rs1051730 (SEQ ID n° 35/36), où une présence homozygote du SNP rs6495308-C/C (SEQ ID n° 3), une présence hétérozygote d'un SNP rs6495308-C/T, ou une présence homozygote du génotype de SNP rs1051730-C/C (SEQ ID n° 35) constitue une indication selon laquelle l'individu répondra probablement à un traitement par un agoniste du récepteur nicotinique alpha-7 de l'acétylcholine, et
où l'agoniste du récepteur nicotinique alpha-7 de l'acétylcholine est le (R)-3-(6-p-tolylpyridin-3-yloxy)-1-azabicyclo[2.2.2]octane sous forme de base libre ou sous forme de sel d'addition d'acide.

13. Utilisation d'au moins une sonde destinée à la détection
(i) du SNP rs55853698-T/T (SEQ ID n° 1) de *CHRNA5,* ou
(ii) du SNP rs6495308-C/T ou -C/C (SEQ ID n° 3/4) de *CHRNA3,* ou
(iii) du SNP rs1051730-C/C (SEQ ID n° 35/36) de *CHRNA3,* ou
(iv) un SNP formant un haplotype avec lesdits SNP, afin de déterminer si un individu peut répondre
(a) vis-à-vis du traitement d'un trouble cognitif, ou d'un trouble psychotique et/ou neurodégénératif par le (R)-3-(6-p-tolylpyridin-3-yloxy)-1-azabicyclo-[2.2.2]octane, ou
(b) à l'augmentation des performances cognitives par le (R)-3-(6-p-tolylpyridin-3-yloxy)-1-azabicyclo-[2.2.2]octane.

14. Méthode ou utilisation selon l'une quelconque des revendications 11 à 13, où le trouble cognitif, le trouble psychotique et/ou neurodégénératif est la schizophrénie.
